# EUROPEAN PATENT SPECIFICATION

(11) **EP 2 271 374 B1**
(45) Date of publication and mention of the grant of the patent: **05.03.2014**
(21) Application number: 09724494.1
(22) Date of filing: 25.03.2009
(51) Int. Cl.: A61L 2/00, A61J 1/10

(54) **METHOD OF VIRAL INACTIVATION OF BIOLOGICAL FLUIDS BY SOLVENT/DETERGENT-TREATMENT**
VERFAHREN ZUR VIRALEN INAKTIVIERUNG BIOLOGISCHER FLÜSSIGKEITEN MITTELS LÖSUNGS-/REINIGUNGSMITTELBEHANDLUNG
PROCÉDÉ D INACTIVATION VIRALE DE FLUIDES BIOLOGIQUES PAR TRAITEMENT AVEC UN SOLVANT/DÉTERGENT

(30) Priority: 28.03.2008 US 40180
(43) Date of publication of application: 12.01.2011
(73) Proprietor: Research Foundation For Medical Devices, 1705 Fribourg (CH)
(72) Inventor: BURNOUF, Thierry, 59000 Lill (FR); EL-EKIABY, Magdy, Cairo 11431 (EG); GOUBRAN, Hadi Alphonse, Cairo 11431 (EG); RADOSEVICH, Miryana, F-59249 Aubers (FR)
(74) Representative: Cabinet Plasseraud
(86) International application number: PCT/EP2009/053491
(87) International publication number: WO 2009/118331

(56) References cited:
- WO-A-2005/082937
- WO-A-2006/082115
- US-A- 5 834 420
- US-A1- 2006 234 907
- US-A1- 2007 173 638
- US-B1- 7 112 320

## Description

The present invention relates to viral inactivation of biological fluids, in particular of blood plasma and blood plasma products.

Human and animal blood plasma and plasma fractions play an important role in modern medicine. They are mainly used for transfusion purposes in the treatment of bleeding episodes, thrombotic episodes, fibrinolysis, infectious episodes, or for prophylaxis or for pretreating patients prior to surgery in certain clinical situations.

A major drawback in the use of human or animal blood plasma or plasma fractions is the risk of transmission of blood borne viruses, such as human immunodeficiency virus (HIV), hepatitis B virus (HBV), hepatitis C virus (HCV), and West Nile Virus, or possibly emerging viruses such as Dengue virus, shikungunya virus, Severe Acute Respiratory Syndrome (SARS) virus, and avian flu virus, which are all lipid-enveloped viruses. Viral risks also exist from products obtained from recombinant mammalian cell lines and from transgenic animals.

Therefore, different methods for viral inactivation of blood plasma and/or plasma fractions and/or recombinant products have been developed, such as the treatment with methylene blue followed by irradiation with visible light and solvent/detergent or solvent alone treatment. US patent Nos. 4,481,189 and 4,540,573 describe for example the use of organic solvent detergent combinations (S/D method) or solvent alone to reduce by several orders of magnitude the infectivity of hepatitis viruses or other enveloped viruses contained in plasma and plasma products or added thereto.

Solvent/detergent or solvent alone treatment under appropriate conditions of reagent concentration, temperature and contact time effectively disassembles viruses that have envelope proteins associated with lipids, while having negligible effect on the molecular conformations and biological activity of sensitive plasma proteins.

Today the solvent/detergent (S/D) method is the main viral inactivation treatment of many plasma and biotech (recombinant or transgenic products derived from mammalian cells or mammals) products on the market, including plasma for transfusion. The procedure generally consists in incubating protein solutions for 1 to 6 hours with 0.3 to 1% by volume with respect to the volume of the protein solution of an organic solvent, tri(n-butyl) phosphate (TnBP), and of one or several detergents, generally Tween-80, Triton X-100, Tween-20, or sodium deoxycholate. The plasma or plasma products are treated in large pools of a large number of donations (100 to 5000litres, or more), which requires large and expensive pharmaceutical manufacturing facilities.

The treatment preserves the functional activities of a wide range of coagulation factors and other labile plasma proteins with an excellent record of virucidal efficiency for the pathogenic lipid-enveloped blood-borne viruses (Burnouf and Radosevich, Blood Rev 2000, 14: 94-110).

Still, after the SD treatment, large-pool SD (LP/SD) plasma for transfusion is subjected to hydrophobic interaction chromatography in order to remove the SD agents. However, this hydrophobic interaction chromatography step involves expensive chromatographic materials, relatively large buffer consumption and requires cleaning and sanitization of the equipment after each batch, since the equipment is reused, thus rendering the SD removal rather expensive and lengthy. Furthermore, due to the reuse of the equipment there is a risk of batch to batch contamination by viruses and prions that tend to stick to the surfaces of the equipment.

Moreover, large-pool SD (LP/SD) plasma for transfusion subjected to incubation with 1 volume% of TnBP with respect to the weight of the biological fluid and 1 weight% of Triton X-100 (polyoxyethylene (9-10) p-t-octyl phenol; molecular formula t-Oct-C₆H₄-(OCH₂CH₂)ₓ, x=9-10) with respect to the weight of the biological fluid, oil extraction, centrifugation, and hydrophobic interaction chromatography as for example described in Horowitz B. et al., Blood 1992; 79: 826-31, has low functional activity of certain serine protease inhibitors (serpins), such as alpha 2-antiplasmin (α2-AP or SERPINF2), or anticoagulants such as Protein S.

International patent application WO 2006/082115 discloses in one of the examples the viral inactivation of plasma with 1% TnBP and 1% Triton X-45, followed by one, two or three castor oil extractions. As shown in the Example section of the present application, the obtained biological fluid is turbid and cannot be filtered by gravity over activated charcoal.

US patent no. 7,112,320 discloses a method for producing a wound healing promoter delivery system comprising the step of preparing a concentrated solution of fibronectin adjusted to pH of 8.0 to 11.6. Said solution of fibronectin is obtained from cryoprecipitate which is virally inactivated by the solvent/detergent (S/D) procedure using TnBP and Triton X-100 (polyoxyethylene (9-10) p-t-octyl-phenol of formula t-Oct-C₆H₄-(OCH₂CH₂)ₓOH, x= 9-10) followed by a soybean oil extraction.

US patent application 2006/234907 A1 relates to a process for the preparation of albumin in which the albumin is virally inactivated by the SD method using TnBP and Triton X-100 followed by a castor oil extraction.

As demonstrated in the Example section of the present application, biological fluids that are submitted to an S/D treatment with TnBP and Triton X-100 followed by castor oil extraction are turbid and cannot be filtered by gravity over activated charcoal.

In view of the above drawbacks of the prior art, there is a need for a viral inactivation method using SD treatment that does not necessitate hydrophobic interaction chromatography in order to satisfactorily remove the SD chemicals and that has high functional activity of α2-AP and/or Protein S.

After long and intensive research, the inventors have now found surprisingly and unexpectedly that this aim is reached by a method of virally inactivating a biological fluid as defined in claim 1 comprising:
(a) a step of treating the biological fluid with a mixture of tri(n-butyl)phosphate (TnBP) and polyoxyethylene (4-5) p-t-octyl-phenol (t-Oct-C₆H₄-(OCH₂CH₂)ₓOH, x ≈ 5; Triton X-45) ;
(b) an oil extraction step, wherein the tri(n-butyl)phosphate and polyoxyethylene (4-5) p-t-octyl-phenol (t-Oct-C₆H₄-(OCH₂CH₂)ₓOH, x ≈ 5) containing biological fluid is treated with 5 to 15 %, preferably 8 to 12%, and even more preferably about 10 % by volume of soybean oil so as to transfer the tri(n-butyl)phosphate and polyoxyethylene (4-5) p-t-octyl-phenol (t-Oct-C₆H₄-(OCH₂CH₂)ₓOH, x ≈ 5) to the soybean oil;
(c) passing the biological fluid recovered after step (b) through an activated charcoal filter by gravity;
(d) passing the biological fluid recovered after step (c) through a bacterial filter by gravity;
provided that the soybean oil extraction step is the sole oil extraction step of the method.

The tri(n-butyl)phosphate (TnBP) is advantageously used in an amount from 0.1 to 2 volume% with respect to the volume of the biological fluid, preferably 0.3 to 1.5 volume%, and even more preferably 0.5 to 1 volume%, for example 1 volume%.

Advantageously, the polyoxyethylene (4-5) p-t-octyl phenol (t-Oct-C₆H₄-(OCH₂CH₂)ₓOH, x= ∼5) (Triton x-45) is used in an amount from 0.1 to 2 volume% with respect to the volume of the biological fluid, preferably 0.3 to 1.5 volume%, and even more preferably 0.5 to 1 volume%, for example 1 volume%.

In a preferred embodiment of the invention, each of the tri(n-butyl)phosphate and the polyoxyethylene (4-5) p-t-octyl-phenol (t-Oct-C₆H₄-(OCH₂CH₂)ₓOH, x ≈ 5) is used in an amount of 1 volume% with respect to the volume of the biological fluid.

The biological fluid according to the present invention is selected from the group consisting of mammalian blood, blood plasma, blood serum, plasma fractions, precipitates from blood fractions and supernatants from blood fractionation, platelet poor plasma, platelet-rich plasma, cryo-poor plasma (cryosupernatant), recombinant and transgenic products. Preferred biological fluids are blood plasma, including recovered plasma (plasma from whole blood) and apheresis plasma, cryo-poor plasma, plasma fractions, such as fractions for the purification of prothrombin complex, Factor IX, Factor VII, Protein C, Protein S, Antithrombin, α2-AP, alpha 1-antitrypsin (α1-AT), plasminogen activator inhibitor-1 (PAI-1), C1-inhibitor, precipitates from plasma, such as polyethylene glycol, caprylic acid, or ammonium sulphate precipitated fractions, and their corresponding supernatants, or any other precipitates or supernatants from plasma fractionation, supernatant I+II+III, precipitate I+II+III, supernatant II+III, precipitate II+III, supernatant I+III, supernatant IV, precipitate V, or precipitate II. Plasma fractions, precipitates and supernatants may be obtained from recovered plasma (plasma from whole blood) as well as from apheresis plasma.

Particularly preferred biological fluids are apheresis plasma, recovered plasma, cryoprecipitate from apheresis plasma, cryoprecipitate from recovered plasma, and cryo-poor plasma (apheresis and recovered).

The performance of one extraction with soybean oil after the SD treatment of step (a) allows to reduce the TnBP content to less than 10 % of the initial TnBP content (10'000 ppm), i.e. to less than about 1000 ppm, preferably to less than 5% of the initial TnBP content (10'000 ppm), i.e. to less than about 500 ppm, and (b) reduce the Triton X-45 content to less than 30% of the initial Triton X-45 content (10'000 ppm), i.e. to less than 3000 ppm, preferably to less than 25% of the initial Triton X-45 content (10'000 ppm), i.e. to less than 2500 ppm.

Moreover, very unexpectedly and surprisingly, the di(2-ethylhexyl) phthalate (DEHP) content in the virally inactivated biological fluid obtained according to the method of the invention is significantly lower than in the starting biological fluid. The DEHP in the starting biological fluid results from the diffusion of DEHP contained in the material of the storage bags commonly used for biological fluids (e.g. plasma, cryo-poor plasma, or cryoprecipitate). Storage and collection bags for biological fluids, such as blood and blood components, are usually made of PVC (polyvinylchloride). DEHP is a plasticizer not chemically bound to the PVC polymer and may leach when PVC medical devices come into contact with fluids. As there are concerns about possible toxicity in special patient groups, like neonates, children, pregnant women, or patients with impaired liver function, there is a need for biological fluids, in particular for transfusion, that have the lowest DEHP content possible. Unexpectedly and surprisingly, both the oil extraction and the filtrations were found to decrease DEHP to levels lower than that of the starting plasma and less than what has been found in plasma for transfusion and platelet concentrates by other groups (see e.g. Inoue, K. etal. Clin. Chem. Acta 2005; 358 (1-2):159-66; Cole RS et al. Vox Sang 1981; 40(5):317-22; Loff S et al. J. Pediatr. Surg. 2000 ; 35 (12) :1775-81 ; Buchta C. et al. Transfusion 2005 ; 45(5) :798-802.

Most importantly the soybean oil extraction is required to obtain a clear / non turbid biological fluid solution that can be filtered without further additional processing (such as centrifugation), therefore making direct efficient filtration possible. Very surprisingly and unexpectedly, a clear / non turbid solution is only obtained when the method comprises only one single soybean oil extraction step. More than one soybean oil extraction or extraction with other oils, such as castor oil always result in a turbid solution that cannot be filtered by gravity through an activated charcoal filter.

Hence, in a preferred embodiment, the filtration steps after the soybean oil extraction, in particular the filtration through an activated charcoal filter may be carried out by gravity.

The charcoal filtration step (c) allows to further reduce the TnBP content to less than 10 ppm or to undetectable level, reduce the Triton X-45 content to less than 50 ppm, or even less than 10ppm, remove oil droplets that may still be present in the biological fluid solution as a result of the mixing during oil extraction, and further clarify the biological fluid solution by removing cell debris, microscopic aggregates, residual lipids, therefore increasing the capacity of the subsequent bacterial filter and making it more readily possible to perform a viral/prion removal step (e.g. to remove non-enveloped viruses) by filtration and/or nanofiltration using adsorption filters such as Cuno Zeta Plus VR filter, or filters with a pore size in the range of 15-75 nm, or equivalent, such as the Pall DV filter, the Millipore Viresolve or, the Asahi Planova filter series.

Examples of suitable activated charcoal filters for filtration of small pools of biological fluids, i.e. less than about 500 ml include, but are not limited to, Pall Supracap 60 filter capsule series such as AKS6, AKS7, AKS4, and AKS1 commercialised by PALL Corporation; and Cuno Zeta Plus ZetaCarbon series such as BIOCAP 30 - R32SP, BIOCAP 30 - R33SP , BIOCAP 30 - R34SP, BIOCAP 30 R35SP, and the newly introduced BIOCAP 25 R32SP, BIOCAP 30 - R33SP , BIOCAP 30 - R34SP, and BIOCAP 25 R35SP commercialized by Cuno Inc..

In an advantageous embodiment of the invention, the bacterial filter comprises pores of a size of about 0.2 µm. The best results with respect to capacity of the bacterial filtration step (d) are obtained when using a bacterial filter having a graduation in its porosity, e.g. from 0.65 µm to 0.2 µm. Such a gradual porosity ensures progressive filtration of the material on fibres of increasingly small porosity. An example of a suitable bacterial filter for filtration of small pools of biological fluids, i.e. less than 1.5 L is the Capsule Mini Kleenpak™ EKV (reference KM5EKVP2S) commercialized by PALL Corporation.

In a preferred embodiment the bacterial filtration in step (d) is carried out by gravity.

Due to the bacterial filtration the bacterial safety of the biological fluid solution is ensured, in the hypothesis adventitious contamination with bacteria would have taken place during processing. Moreover, sterilization of the various chemicals used during processing such as TnBP, Triton X-45, or soybean oil, is avoided, which saves time and costs and renders the method applicable in environments in which sterilization equipment is not readily available.

In the case where the biological fluid is plasma, cryoprecipitate, or cryo-poor plasma, bacterial filtration further ensures the removal of any blood-born bacteria introduced at the time of blood/plasma collection (such as due to improper cleaning of the donor's arm) and which would have not been destroyed by the SD treatment. Moreover, bacterial filtration increases the margin of safety of the plasma/cryoprecipitate/cryo-poor plasma solution with regards to blood-born parasites (e.g. Babesia microti responsible for babesiosis; Plasmodium responsible for malaria; Leishmania responsible for leishmaniosis; Trypanosomia cruzi responsible for Chagas disease) especially in the hypothesis very fresh and unfrozen plasma is used as starting material and they have unexpectedly not been destroyed by the SD treatment. Bacterial filtration on 0.2µm membrane also allows removing blood cell debris, thus reducing the risks of immunological blood transfusion reactions due to residual lymphocytes or cell aggregates.

Moreover, to the knowledge of the inventors, this is the first time that filtration of plasma for transfusion or other plasma components, such as cryoprecipitate or cryo-poor plasma, manufactured by blood establishments through a 0.2 µm filter is possible. The filtration through a filter having pores of a size of about 0.2 µm has the advantage of resulting in products that are free of any blood cells (leucocytes, platelets, red cells) and cell debris, in addition of being free of bacteria and parasites. The complete removal of blood cells and cell debris can also contribute to an improved safety of plasma for transfusion or other plasma components, such as cryoprecipitate or cryo-poor plasma, thanks to removal or a reduction of leucocytes associated pathogens, such as cytomegalovirus, Epstein-Barr virus, or prions. In contrast to the present invention, normal single donor plasma is processed aseptically and contains significant levels of blood cells; leucoreduced single-donor plasma contains low levels of leucocytes but may still contain platelets; virally-inactivated plasma units such as single-donor methylene-blue plasma, or psoralen plasma are run through methylene blue or psoralen adsorption filters, respectively, but still contain blood cells or blood cell debris.

In addition, the plasma and cryo-poor plasma obtained according to the present invention, have a reduced content in lipids, which may reduce the risk of TRALI (Transfusion-Related Acute Lung Injury).

Finally, the plasma and cryoprecipitate obtained according to the present invention, show good preservation of the FVIII and clottable fibrinogen content, while 20 to 30% loss occurs with methylene-blue-, psoralen-, and riboflavin- treated plasma.

In conclusion, the method of the invention allows the preparation of plasma, cryo-poor plasma, and cryoprecipitate that (a) is virally-inactivated, (b) has good preservation of FVIII and clottable fibrinogen, (c) is essentially free of lipids, (d) is essentially free of blood cells and cell debris, and (e) has a lower Di(2-ethylhexyl) phthalate (DEHP) content than the starting plasma, cryo-poor plasma, or cryoprecipitate.

In an advantageous embodiment, the method of the invention comprises, before step (c), a step of passing the biological fluid recovered after step (b) through a transfusion filter by gravity. This additional filtration step does not impact the turbidity of the biological fluid but removes visible clots that might potentially be present in the biological fluid subsequent to soybean oil extraction and thus serves as a safety measure. Preferably, this filtration step is carried out by gravity.

The method of the invention may be applied to industrial processing of large pools of SD treated biological fluids, such as plasma and plasma products, as well as to the processing of so-called minipools (MD-SD). A solvent/detergent method applied to minipools of biological fluids, such as plasma and plasma products (MP/SD), in which small quantities of plasma are treated in a closed bag system, is described in international patent application WO 2006/082115, which is herewith incorporated by reference.

With regard to the in-bag viral inactivation system, the method of the invention is of particular interest since filtration can take place by gravity, without the need of special equipment such as peristaltic pump, thus making it user friendly and inexpensive. The use of disposable filters renders the method particular compatible with the in-bag system since the whole system of bags and filters may simply be discarded after use.
Figure 1 is a schematic drawing of an in-bag system for SD treatment of a plasma (or cryo-poor plasma) mini-pool according to one embodiment of the present invention.
Figure 2 is a schematic drawing of an in-bag system for SD treatment of a cryoprecipitate mini-pool according to one embodiment of the present invention.
Figure 3A is a photograph of an electrophoretic pattern under unreduced conditions of plasma and cryoprecipitate minipools before and after S/D-F treatment (solvent/detergent treatment followed by soybean oil extraction and filtrations).
Figure 3B is a photograph of an electrophoretic pattern under reduced conditions of plasma and cryoprecipitate minipools before and after S/D-F treatment (solvent/detergent treatment followed by soybean oil extraction and filtrations).
Figure 4 shows the mean content in TnBP after S/D treatment, after oil extraction and after filtrations of apheresis and recovered plasma according to Example 20.
Figure 5 shows the mean content in Triton X-45 after S/D treatment, after oil extraction and after filtrations of apheresis and recovered plasma according to Example 20.
Figure 6 illustrates the results of DEHP determination along the various steps of the processing of apheresis plasma or recovered plasma according to Example 20.
Figure 7 shows the VWF multimeric profile of cryoprecipitate minipools before and after the S/D-F treatment according to the invention.

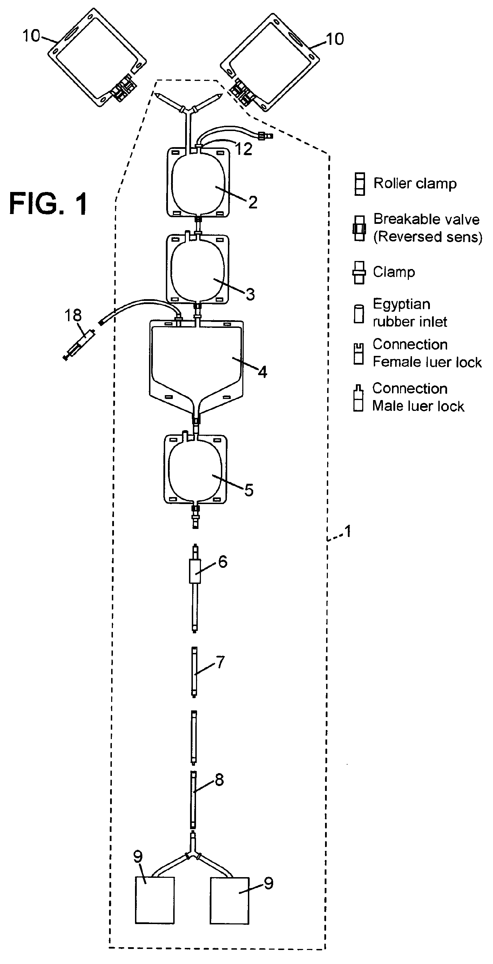

The in-bag system (1) shown in figure 1 represents one particular configuration of a system that is suitable for treating plasma (or cryo-poor plasma) mini-pools according to the method of the invention. This system comprises a first viral inactivation bag (2), a second viral inactivation bag (3), funnel shaped bag (4), a recovery bag (5), a transfusion filter (6), an activated charcoal filter (7), a bacterial filter (8), and two collection bags (9) for recovering the virally inactivated and purified plasma or cryo-poor plasma. In an alternative embodiment, the system does not comprise a transfusion filter (6). The in-bag system may also comprise a further collection bag between the bacterial filter and collection bags (9) to pool the virally inactivated and purified plasma before distribution into collection bags (9).

In an advantageous embodiment, the viral inactivation bags are oval or round shaped to facilitate the mixing of the components and avoid the presence of dead points where the plasma material would not be in contact with the S/D agents.

The viral inactivation bags (2, 3), funnel shaped bag (4), recovery bag (5), the filters (6, 7, 8), and the collection bags (9) for recovering the virally inactivated and purified plasma or cryo-poor plasma are connected to one another in line via state of the art connections used in the field of blood and blood product transfusion.

The plasma or cryo-poor plasma that is to be treated is directly transferred from one or more plasma collection or storage bags (10) to a first inlet (11) of the first viral in activation bag (2). The process chemicals (triton X-45 and TnBP) are then added to the first bag (2) via a second inlet (12), for example by means of a syringe. The syringe may either be connected directly or by means of a tube to the second inlet.

After a first incubation in the first viral inactivation bag (2), the process chemicals containing plasma or cryo-poor plasma is transferred to the second viral inactivation bag (3) for further incubation via outlet (13) of the first bag (2) and inlet (14) of the second bag (3).

After termination of the second incubation step, the process chemicals containing plasma or cryo-poor plasma is transferred from the second viral inactivation bag (3) to the funnel-shaped bag (4) for oil extraction via outlet (15) of the second bag (3) and first inlet (16) of the funnel-shaped bag (4).

The soybean oil for the oil extraction is added via a second inlet (17) of the funnel-shaped bag (4), for example by means of a syringe (18). The syringe (18) may either be connected directly or by means of a tube to the second inlet.

During the oil extraction step, the process chemicals containing plasma or cryo-poor plasma and the soybean oil are first well mixed and then submitted to phase separation by gravity.

After termination of the phase separation by gravity, a lower plasma or cryo-poor plasma phase and an upper soybean oil phase are obtained. The lower phase is then transferred to recovery bag (5) via outlet (19) of the funnel-shaped bag (4) and inlet (20) of the recovery bag (5).

The recovered plasma or cryo-poor plasma then is successively passed through transfusion filter (6) (if present), activated charcoal filter (7) and bacterial filter (8). After the passage through bacterial filter (8), the virally inactivated and purified plasma or cryo-poor plasma is recovered in collection bags (9).

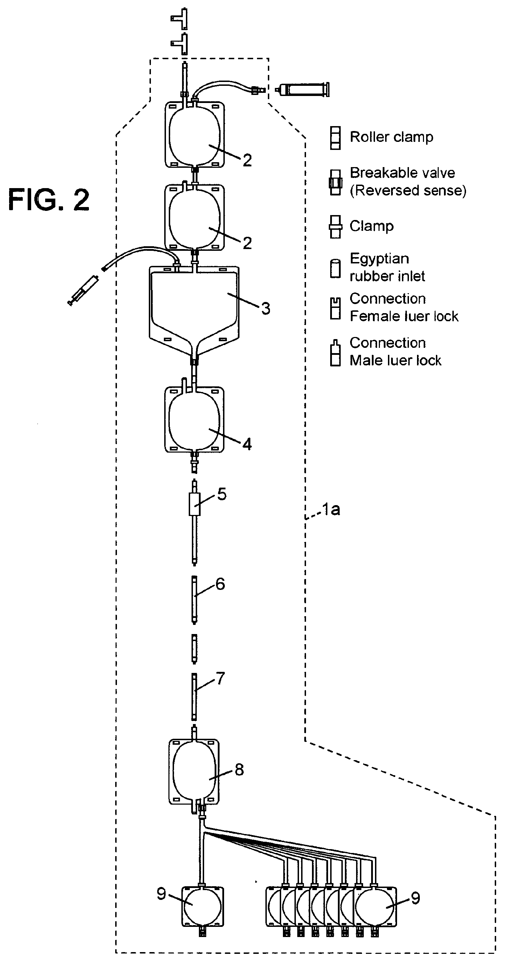

The in-bag system (1a) shown in figure 2 represents one particular configuration of a system that is suitable for treating cryoprecipitate mini-pools according to the method of the invention. This system differs from the system of figure 1 in that it further comprises a further collection bag to mix the treated cryoprecipitate and get an homogeneous solution prior to dispensing (21) between the bacterial filter (8) and collection bags (9). In an alternative embodiment, the system does not comprise a transfusion filter (6).

In the case of viral inactivation of cryoprecipitate in an in-bag system (1a) as shown in figure 2, 30-50 units of (about 10 ml/unit) are directly transferred from their storage bags to a first inlet (11) of the first viral in activation bag (2).

The subsequent procedure is essentially the same than the one for plasma or cryo-poor plasma.

As applied to industrial processing of large pools of SD-treated biological fluids, in particular plasma and fractionated plasma products, such as immunoglobulins G, the described production sequence allows improvements in yield and simplifying the manufacturing process in more than one way. First, the expensive hydrophobic interaction step (usually C18 or SDR-hyper D packing material) to remove the SD agents used at industrial scale is replaced by a single-use charcoal filter. This avoids requirement for validation, cleaning and sanitization of the chromatographic material (which is reused), and reduces/avoids buffer consumptions, making the process less expensive and faster, and avoids the concerns for batch to batch contamination by viruses and prions that tend to stick to the surfaces of the chromatography equipment. Second, the use of Triton X-45 which is needed to get a clear material after the soybean oil provides better quality plasma with good content in α2-AP, α1-AT, Protein S (PS), and other protease inhibitors or anticoagulants.

Another object of the invention is a virally-inactivated biological fluid selected from the group consisting of plasma, cryo-poor plasma, and cryoprecipitate, wherein the biological fluid is essentially free of lipids, free of blood cells and cell debris. The biological fluids, namely plasma and cryoprecipitate, have advantageously good preservation of FVIII and clottable fibrinogen.

The absence of lipids, blood cells and cell debris in the biological fluid can significantly reduce the risk of immunological blood transfusion reactions, such as TRALI (Transfusion-Related Acute Lung Injury) due to residual lipids, or lymphocytes or cell aggregates. It can also reduce the risk from pathogens that associated to blood cells or blood cell membranes.

In order to more fully illustrate the nature of the invention and the manner of practising the same, the following non-limiting examples are presented. Although all the examples involve mini-pools and are carried out in a closed bag system, it should be understood that the method may easily be up scaled, especially in respect of filtration through activated charcoal and bacterial filters. Suitable large scale filters are commercially available.

### [EXAMPLES]

Throughout the examples all percentages are volume percentages, unless otherwise specified.

### Example 1:

### Apheresis plasma treated with 1 % TaBP-1 % Triton X-45 (reference 93419, Fluka, Sigma Aldrich) and subjected to 1 extraction by soybean oil (reference S7381, Sigma)

2 plasma units were collected by apheresis procedures following manufacturers' instructions and anticoagulated with ACDA (ratio: 8ml/100 ml of blood). Plasma was not leucoreduced prior to processing. Plasma was frozen in a dry ice ethanol bath and stored in a freezer at -30°C for a maximum of 3 months.

The 2 plasma donations were thawed in a temperature-controlled water-bath at 35°C for 10 min and then pooled into the first SD treatment bag. The volume of the plasma pool was 400ml.

8 ml of a 50%/50% mixture of TnBP (Prolabo, VWR, Fontenay-sous-bois, France) and Triton X-45 (reference 93419, Fluka, Sigma Aldrich) was carefully added to the 400 ml of plasma to reach final concentrations of 1% TnBP and 1% Triton X-45 with respect to the weight of the plasma.

The plasma-TnBP-Triton X-45 mixture was shaken vigorously for 5 min to ensure good mixing of the various components. The processing bag was then incubated in a shaker-incubator at 31°C and then treated for 30 minutes under constant gentle stirring (150 rpm). The plasma-TnBP-Triton X-45 mixture was then transferred into the second SD treatment bag and incubated in the same conditions for 1h30min.

The mixture was then transferred to the oil extraction bag. 40 ml of soybean oil (reference S7381, Sigma) was added to the plasma-TnBP-Triton X-45 mixture. The plasma-TnBP-Triton X-45/ 10 vol% oil suspension was shaken vigorously for at least 1 min and then put onto the shaker-incubator to ensure gentle stirring for at least 15 min. The bag was then suspended to ensure 45 minutes decantation between the oil phase (upper layer) and the plasma phase (lower layer).

A clear plasma phase was recovered. Over 370ml of plasma passed through an infusion filter and then could be filtered by gravity (without pressure applied by a pump) through a AKS6 Pall charcoal filter (SUPRAcap 60 SC060XAK6) connected in series to a 0.2µm bacterial filter from Pall (Mini kleenpak™ 20, réf. KM5EKVP2S).

Conclusion: the combination of the use of 1% TnBP-1% Triton X-45 followed by 1 extraction by soybean oil allows to obtain over 350 ml of clear plasma that can be filtered by gravity through a charcoal filter and a bacterial filter to eliminate the SD agents, oil residues, blood cell debris, bacteria and parasites.

### Example 2:

### Apheresis plasma treated with 1% TnBP-1% Triton X-45 (reference 93419, Fluka, Sigma Aldrich) and subjected to 1 extraction by soybean oil (ref. 85471, from Glycine max, Sigma)

2 plasma units were collected by apheresis procedures following manufacturers' instructions and anticoagulated with ACDA (ratio: 8ml/100 ml of blood). Plasma was not leucoreduced prior to processing. Plasma was frozen in a dry ice ethanol bath and stored in a freezer at -30°C for a maximum of 3 months.

The 2 plasma donations were thawed in a temperature-controlled water-bath at 35°C for 10 min and then pooled into the first SD treatment bag. The volume of the plasma pool was 400ml.

8 ml of a 50%/50% mixture of TnBP (Prolabo, VWR, Fontenay-sous-bois, France) and Triton X-45 (reference 93419, Fluka, Sigma Aldrich) was carefully added to the 400 ml of plasma to reach final concentrations of 1% TnBP and 1% Triton X-45.

The plasma-TnBP-Triton X-45 mixture was shaken vigorously for 5 min to ensure good mixing of the various components. The processing bag was then incubated in a shaker-incubator at 31°C and then treated for 30 minutes under constant gentle stirring (150 rpm). The plasma-TnBP-Triton X-45 mixture was then transferred into the second SD treatment bag and incubated in the same conditions for 1h30min.

The mixture was then transferred to the oil extraction bag. 40 ml of soybean oil (ref. 85471, from Glycine max, Sigma) was added to the plasma-TnBP-Triton X-45 mixture. The plasma-TnBP-Triton X-45/ 10 vol% oil suspension was shaken vigorously for at least 1 min and then put onto the shaker-incubator to ensure gentle stirring for at least 15 min. The bag was then suspended to ensure 45 minutes decantation between the oil phase (upper layer) and the plasma phase (lower layer).

A clear plasma phase was recovered. Over 370ml of plasma passed through an infusion filter and then could be filtered by gravity (without pressure applied by a pump) through a AKS6 Pall charcoal filter (SUPRAcap 60 SC060XAK6) connected in series to a 0.2µm bacterial filter from Pall (Mini kleenpak™ 20, réf. KM5EKVP2S).

Conclusion: the combination of the use of 1% TnBP-1% Triton X-45 followed by 1 extraction by soybean oil allows obtaining over 350 ml of clear plasma that can be filtered by gravity through a charcoal filter and a bacterial filter to eliminate the SD agents, oil residues, blood cell debris, bacteria and parasites.

### Example 3:

### Apheresis plasma treated with 1% TnBP-1% Triton X-45 (reference 93419, Fluka, Sigma Aldrich) and subjected to 1 extraction by soybean oil (ref. 19-42500, Penta International Corporation)

2 plasma units were collected by apheresis procedures following manufacturers' instructions and anticoagulated with ACDA (ratio: 8ml/100 ml of blood). Plasma was not leucoreduced prior to processing. Plasma was frozen in a dry ice ethanol bath and stored in a freezer at -30°C for a maximum of 3 months.

The 2 plasma donations were thawed in a temperature-controlled water-bath at 35°C for 10 min and then pooled into the first SD treatment bag. The volume of the plasma pool was 400ml.

8 ml of a 50%/50% mixture of TnBP (Prolabo, VWR, Fontenay-sous-bois, France) and Triton X-45 (reference 93419, Fluka, Sigma Aldrich) was carefully added to the 400 ml of plasma to reach final concentrations of 1% TnBP and 1% Triton X-45.

The plasma-TnBP-Triton X-45 mixture was shaken vigorously for 5 min to ensure good mixing of the various components. The processing bag was then incubated in a shaker-incubator at 31°C and then treated for 30 minutes under constant gentle stirring (150 rpm). The plasma-TnBP-Triton X-45 mixture was then transferred into the second SD treatment bag and incubated in the same conditions for 1h30min.

The mixture was then transferred to the oil extraction bag. 40 ml of soybean oil (ref. 19-42500, Penta International Corporation, USA) was added to the plasma-TnBP-Triton X-45 mixture. The plasma-TnBP-Triton X-45/ 10 vol% oil suspension was shaken vigorously for at least 1 min and then put onto the shaker-incubator to ensure gentle stirring for at least 15 min. The bag was then suspended to ensure 45 minutes decantation between the oil phase (upper layer) and the plasma phase (lower layer).

A clear plasma phase was recovered. Over 370ml of plasma passed through an infusion filter and then could be filtered by gravity (without pressure applied by a pump) through a AKS6 Pall charcoal filter (SUPRAcap 60 SC060XAK6) connected in series to a 0.2µn bacterial filter from Pall (Mini kleenpak™ 20, réf. KM5EKVP2S).

Conclusion: the combination of the use of 1% TnBP-1% Triton X-45 followed by 1 extraction by soybean oil allows obtaining over 350 ml of clear plasma that can be filtered by gravity through a charcoal filter and a bacterial filter to eliminate the SD agents, oil residues, blood cell debris, bacteria and parasites.

### Example 4:

### Apheresis plasma treated with 1% TnBP-1% Triton X-45 (reference 93419, Fluka, Sigma Aldrich) and subjected to 2 extractions by soybean oil (reference S7381, Sigma)

2 plasma units were collected by apheresis procedures following manufacturers' instructions and anticoagulated with ACDA (ratio: 8ml/100 ml of blood). Plasma was not leucoreduced prior to processing. Plasma was frozen in a dry ice ethanol bath and stored in a freezer at -30°C for a maximum of 3 months.

The 2 plasma donations were thawed in a temperature-controlled water-bath at 35°C for 10 min and then pooled into the first SD treatment bag. The volume of the plasma pool was 400ml.

8 ml of a 50%/50% mixture of TnBP (Prolabo, VWR, Fontenay-sous-bois, France) and Triton X-45 (reference 93419, Fluka, Sigma Aldrich) was carefully added to the 400 ml of plasma to reach final concentrations of 1% TnBP and 1% Triton X-45.

The plasma-TnBP-Triton X-45 mixture was shaken vigorously for 5 min to ensure good mixing of the various components. The processing bag was then incubated in a shaker-incubator at 31°C and then treated for 30 minutes under constant gentle stirring (150 rpm). The plasma-TnBP-Triton X-45 mixture was then transferred into the second SD treatment bag and incubated in the same conditions for 1h30min.

The mixture was then transferred to the oil extraction bag. 40 ml of soybean oil (reference S7381, Sigma) was added to the plasma-TnBP-Triton X-45 mixture. The plasma-TnBP-Triton X-45/ 10 vol% oil suspension was shaken vigorously for at least 1 min and then put onto the shaker-incubator to ensure gentle stirring for at least 15 min. The bag was then suspended to ensure 45 minutes decantation between the oil phase (upper layer) and the plasma phase (lower layer).

The plasma phase was recovered and the oil extraction procedure was repeated once, yielding about 360ml of plasma (Total: 2 soybean oil extractions)

A very turbid plasma phase was recovered that could pass through an infusion filter. Only about 30ml could be filtered by gravity through a AKS6 Pall charcoal filter (SUPRAcap 60 SC060XAK6) and the filter started to clog stopping the filtration process.

Conclusion: the combination of the use of 1% TnBP-1% Triton X-45 followed by 2 extractions by soybean oil yields in a very turbid plasma that cannot be filtered by gravity through a charcoal filter and a bacterial filter.

### Example 5:

### Apheresis plasma treated with 1% TnBP-1% Triton X-45 (reference 93419, Fluka, Sigma Aldrich) and subjected to 3 extractions by soybean oil (reference S7381, Sigma)

2 plasma units were collected by apheresis procedures following manufacturers' instructions and anticoagulated with ACDA (ratio: 8ml/100 ml of blood). Plasma was not leucoreduced prior to processing. Plasma was frozen in a dry ice ethanol bath and stored in a freezer at -30°C for a maximum of 3 months.

The 2 plasma donations were thawed in a temperature-controlled water-bath at 35°C for 10 min and then pooled into the first SD treatment bag. The volume of the plasma pool was 400ml.

8 ml of a 50%/50% mixture of TnBP (Prolabo, VWR, Fontenay-sous-bois, France) and Triton X-45 (reference 93419, Fluka, Sigma Aldrich) was carefully added to the 400 ml of plasma to reach final concentrations of 1% TnBP and 1% Triton X-45.

The plasma-TnBP-Triton X-45 mixture was shaken vigorously for 5 min to ensure good mixing of the various components. The processing bag was then incubated in a shaker-incubator at 31°C and then treated for 30 minutes under constant gentle stirring (150 rpm). The plasma-TnBP-Triton X-45 mixture was then transferred into the second SD treatment bag and incubated in the same conditions for 1h30min.

The mixture was then transferred to the oil extraction bag. 40 ml of soybean oil (reference S7381, Sigma) was added to the plasma-TnBP-Triton X-45 mixture. The plasma-TnBP-Triton X-45/ 10 vol% oil suspension was shaken vigorously for at least 1 min and then put onto the shaker-incubator to ensure gentle stirring for at least 15 min. The bag was then suspended to ensure 45 minutes decantation between the oil phase (upper layer) and the plasma phase (lower layer).

The plasma phase was recovered and the oil extraction procedure was repeated twice, yielding about 350ml of plasma.

A slightly turbid plasma phase was recovered. 350ml of plasma passed through an infusion filter. Only about 80ml could be filtered by gravity through a AKS6 Pall charcoal filter (SUPRAcap 60 SC060XAK6) and the filter started to clog stopping the filtration process.

Conclusion: the combination of the use of 1% TnBP-1% Triton X-45 followed by 3 extractions by soybean oil yields in a slightly turbid plasma that cannot be filtered by gravity through a charcoal filter and a bacterial filter.

### Example 6:

### Apheresis plasma treated with 1% TnBP-1% Triton X-45 (X45, Triton® X-45, Sigma) and subjected to 1 extraction by soybean oil (reference S7381, Sigma)

2 plasma units were collected by apheresis procedures following manufacturers' instructions and anticoagulated with ACDA (ratio: 8ml/100 ml of blood). Plasma was not leucoreduced prior to processing. Plasma was frozen in a dry ice ethanol bath and stored in a freezer at -30°C for a maximum of 3 months.

The 2 plasma donations were thawed in a temperature-controlled water-bath at 35°C for 10 min and then pooled into the first SD treatment bag. The volume of the plasma pool was 400ml.

8 ml of a 50%/50% mixture of TnBP (Prolabo, VWR, Fontenay-sous-bois, France) and Triton X-45 (X45, Triton® X-45, Sigma) was carefully added to the 400 ml of plasma to reach final concentrations of 1% TnBP and 1% Triton X-45.

The plasma-TnBP-Triton X-45 mixture was shaken vigorously for 5 min to ensure good mixing of the various components. The processing bag was then incubated in a shaker-incubator at 31°C and then treated for 30 minutes under constant gentle stirring (150 rpm). The plasma-TnBP-Triton X-45 mixture was then transferred into the second SD treatment bag and incubated in the same conditions for 1h30min.

The mixture was then transferred to the oil extraction bag. 40 ml of soybean oil (reference S7381, Sigma) was added to the plasma-TnBP-Triton X-45 mixture. The plasma-TnBP-Triton X-45/ 10 vol% oil suspension was shaken vigorously for at least 1 min and then put onto the shaker-incubator to ensure gentle stirring for at least 15 min. The bag was then suspended to ensure 45 minutes decantation between the oil phase (upper layer) and the plasma phase (lower layer).

A clear plasma phase was recovered. Over 370ml of plasma passed through an infusion filter and then could be filtered by gravity (without pressure applied by a pump) through a AKS6 Pall charcoal filter (SUPRAcap 60 SC060XAK6) connected in series to a 0.2µm bacterial filter from Pall (Mini kleenpak™ 20, réf. KM5EKVP2S).

Conclusion: the combination of the use of 1% TnBP-1% Triton X-45 (using another brand of Triton X-45) followed by 1 extraction by soybean oil allows to obtain over 350 ml of a clear plasma that can be filtered by gravity through a charcoal filter and a bacterial filter to eliminate the SD agents oil residues, blood cell debris, bacteria and parasites.

### Example 7:

### Apheresis plasma treated with 2% TnBP and subjected to 1, 2, or 3 extractions by soybean oil (reference S7381, Sigma)

2 plasma units were collected by apheresis procedures following manufacturers' instructions and anticoagulated with ACDA (ratio: 8ml/100 ml of blood). Plasma was not leucoreduced prior to processing. Plasma was frozen in a dry ice ethanol bath and stored in a freezer at -30°C for a maximum of 3 months.

The 2 plasma donations were thawed in a temperature-controlled water-bath at 35°C for 10 min and then pooled into the first SD treatment bag. The volume of the plasma pool was 400ml.

8 ml of TnBP (Prolabo, VWR, Fontenay-sous-bois, France) was carefully added to the 400 ml of plasma to reach final concentrations of 2% TnBP.

The plasma-TnBP mixture was shaken vigorously for 5 min to ensure good mixing of the various components. The processing bag was then incubated in a shaker-incubator at 37°C and then treated for 30 minutes under constant gentle stirring (150 rpm). The plasma-TnBP mixture was then transferred into the second SD treatment bag and incubated in the same conditions for 1h30min.

The mixture was then transferred to the oil extraction bag. 40 ml of soybean oil (reference S7381, Sigma) was added to the plasma-TnBP mixture. The plasma-TnBP/ 10 vol% oil suspension was shaken vigorously for at least 1 min and then put onto the shaker-incubator to ensure gentle stirring for at least 15 min. The bag was then suspended to ensure 45 minutes decantation between the oil phase (upper layer) and the plasma phase (lower layer).

The plasma phase recovered was very turbid making filtration through a charcoal filter impossible (AKS6 Pall charcoal filter; SUPRAcap 60 SC060XAK6).

The oil extraction procedure was repeated twice. In each case the plasma was very turbid making the above-mentioned filtration step impossible.

Conclusion: the combination of the use of 2% TnBP followed by 1, 2, or 3 extractions by soybean oil yields in each case a very turbid plasma that cannot be filtered by gravity through a AKS6 Pall charcoal filter (SUPRAcap 60 SC060XAK6) and even less through a bacterial filter.

### Example 8:

### Apheresis plasma treated with 1% TnBP-1% Triton X-45 (reference 93419, Fluka, Sigma Aldrich) and subjected to 1, 2, or 3 extractions by castor oil

2 plasma units were collected by apheresis procedures following manufacturers' instructions and anticoagulated with ACDA (ratio: 8ml/100 ml of blood). Plasma was not leucoreduced prior to processing. Plasma was frozen in a dry ice ethanol bath and stored in a freezer at -30°C for a maximum of 3 months.

The 2 plasma donations were thawed in a temperature-controlled water-bath at 35°C for 10 min and then pooled into the first SD treatment bag. The volume of the plasma pool was 400ml.

8 ml of a 50%/50% mixture of TnBP (Prolabo, VWR, Fontenay-sous-bois, France) and Triton X-45 (reference 93419, Fluka, Sigma Aldrich) was carefully added to the 400 ml of plasma to reach final concentrations of 1% TnBP and 1% Triton X-45.

The plasma-TnBP-Triton X-45 mixture was shaken vigorously for 5 min to ensure good mixing of the various components. The processing bag was then incubated in a shaker-incubator at 31°C and then treated for 30 minutes under constant gentle stirring (150 rpm). The plasma-TnBP-Triton X-45 mixture was then transferred into the second SD treatment bag and incubated in the same conditions for 1h30min.

The mixture was then transferred to the oil extraction bag. 40 ml of castor (= ricinus) oil (ref. 83912, Fluka) was added to the plasma-TnBP-Triton X-45 mixture. The plasma-TnBP-Triton X-45/ 10 vol% oil suspension was shaken vigorously for at least 1 min and then put onto the shaker-incubator to ensure gentle stirring for at least 15 min. The bag was then suspended to ensure 45 minutes decantation between the oil phase (upper layer) and the plasma phase (lower layer).

The plasma phase recovered was turbid making filtration through a charcoal filter impossible (AKS6 Pall charcoal filter; SUPRAcap 60 SC060XAK6).

The oil extraction procedure was repeated twice. In each case the plasma was turbid making the above-mentioned filtration step impossible.

Conclusion: the combination of the use of 1% TnBP-1% Triton X-45 followed by 1, 2, or 3 extractions by castor oil yields in turbid plasma that cannot be filtered by gravity through a charcoal filter and a bacterial filter.

### Example 9:

### Apheresis plasma treated with 1% TnBP-1% Triton X-100 and subjected to 1, 2, or 3 extractions by soybean oil (reference S7381, Sigma)

2 plasma units were collected by apheresis procedures following manufacturers' instructions and anticoagulated with ACDA (ratio: 8ml/100 ml of blood). Plasma was not leucoreduced prior to processing. Plasma was frozen in a dry ice ethanol bath and stored in a freezer at -30°C for a maximum of 3 months.

The 2 plasma donations were thawed in a temperature-controlled water-bath at 35°C for 10 min and then pooled into the first SD treatment bag. The volume of the plasma pool was 400ml.

8 ml of a 50%/50% mixture of TnBP (Prolabo, VWR, Fontenay-sous-bois, France) and Triton X-100 (Merck, Darmstadt, Germany) was carefully added to the 400 ml of plasma to reach final concentrations of 1% TnBP and 1% Triton X-100.

The plasma-TnBP-Triton X-100 mixture was shaken vigorously for 5 min to ensure good mixing of the various components. The processing bag was then incubated in a shaker-incubator at 31°C and then treated for 30 minutes under constant gentle stirring (150 rpm). The plasma-TnBP-Triton X-100 mixture was then transferred into the second SD treatment bag and incubated in the same conditions for 1h30min.

The mixture was then transferred to the oil extraction bag. 40 ml of soybean (reference S7381, Sigma) was added to the plasma-TnBP-Triton X-100 mixture. The plasma-TnBP-Triton X-100/ 10 vol% oil suspension was shaken vigorously for at least 1 min and then put onto the shaker-incubator to ensure gentle stirring for at least 15 min. The bag was then suspended to ensure 45 minutes decantation between the oil phase (upper layer) and the plasma phase (lower layer).

The plasma phase recovered was turbid making filtration through a charcoal filter impossible (AKS6 Pall charcoal filter; SUPRAcap 60 SC060XAK6).

The oil extraction procedure was repeated twice. In each case the plasma was turbid making the above-mentioned filtration step impossible.

Conclusion: the combination of the use of 1% TnBP-1% Triton X-100 followed by 1, 2, or 3 extractions by soybean oil yields in turbid plasma that cannot be filtered by gravity through a charcoal filter and a bacterial filter.

### Example 10:

### Apheresis plasma treated with 1% TnBP-1% Tween 80 and subjected to 1, 2, or 3 extractions by soybean oil (reference S7381, Sigma)

2 plasma units were collected by apheresis procedures following manufacturers' instructions and anticoagulated with ACDA (ratio: 8ml/100 ml of blood). Plasma was not leucoreduced prior to processing. Plasma was frozen in a dry ice ethanol bath and stored in a freezer at -30°C for a maximum of 3 months.

The 2 plasma donations were thawed in a temperature-controlled water-bath at 35°C for 10 min and then pooled into the first SD treatment bag. The volume of the plasma pool was +/- 400ml.

8 ml of a 50%/50% mixture of TnBP (Prolabo, VWR, Fontenay-sous-bois, France) and Tween-80, also known as polysorbate-80 (Ref 59924, Fluka) was carefully added to the 400 ml of plasma to reach final concentrations of 1% TnBP and 1% Tween-80.

The plasma-TnBP-Tween-80 mixture was shaken vigorously for 5 min to ensure good mixing of the various components. The processing bag was then incubated in a shaker-incubator at 31°C and then treated for 30 minutes under constant gentle stirring (150 rpm). The plasma-TnBP-Tween-80 mixture was then transferred into the second SD treatment bag and incubated in the same conditions for 1h30min.

The mixture was then transferred to the oil extraction bag. 40 ml of soybean (reference S7381, Sigma) was added to the plasma-TnBP-Tween-80 mixture. The plasma-TnBP-Tween-80/ 10 vol% oil suspension was shaken vigorously for at least 1 min and then put onto the shaker-incubator to ensure gentle stirring for at least 15 min. The bag was then suspended to ensure 45 minutes decantation between the oil phase (upper layer) and the plasma phase (lower layer).

The plasma phase recovered was turbid making filtration through a charcoal filter impossible (AKS6 Pall charcoal filter; SUPRAcap 60 SC060XAK6).

The oil extraction procedure was repeated twice. In each case the plasma was turbid making the above-mentioned filtration step impossible.

Conclusion: the combination of the use of 1% TnBP-1% Tween-80 followed by 1, 2, or 3 extractions by soybean oil yields in turbid plasma that cannot be filtered by gravity through a charcoal filter and a bacterial filter.

### Example 11:

### Recovered plasma treated with 1% TnBP-1% Triton X-45 (reference 93419, Fluka, Sigma Aldrich) and subjected to 1 extraction by soybean oil (reference S7381, Sigma)

2 units of recovered plasma were prepared from whole blood, collected into CPD anticoagulant/preservative (ratio: 14ml/100 ml of blood). Blood was centrifuged at 3600 g for 12 min within 4hrs of collection.Plasma was not leucoreduced prior to processing. Plasma was frozen in a dry ice ethanol bath and stored in a freezer at-30°C for a maximum of 3 months.

The 2 plasma donations were thawed in a temperature-controlled water-bath at 35°C for 10 min and then pooled into the first SD treatment bag. The volume of the plasma pool was 400ml.

8 ml of a 50%/50% mixture of TnBP (Prolabo, VWR, Fontenay-sous-bois, France) and Triton X-45 (reference 93419, Fluka, Sigma Aldrich) was carefully added to the 400 ml of plasma to reach final concentrations of 1% TnBP and 1% Triton X-45.

The plasma-TnBP-Triton X-45 mixture was shaken vigorously for 5 min to ensure good mixing of the various components. The processing bag was then incubated in a shaker-incubator at 31°C and then treated for 30 minutes under constant gentle stirring (150 rpm). The plasma-TnBP-Triton X-45 mixture was then transferred into the second SD treatment bag and incubated in the same conditions for 1h30min.

The mixture was then transferred to the oil extraction bag. 40 ml of soybean oil (reference S7381, Sigma) was added to the plasma-TnBP-Triton X-45 mixture. The plasma-TnBP-Triton X-45/ 10 vol% oil suspension was shaken vigorously for at least 1 min and then put onto the shaker-incubator to ensure gentle stirring for at least 15 min. The bag was then suspended to ensure 45 minutes decantation between the oil phase (upper layer) and the plasma phase (lower layer).

A clear plasma phase was recovered. Over 370ml of plasma passed through an infusion filter and then could be filtered by gravity (without pressure applied by a pump) through a AKS6 Pall charcoal filter (SUPRAcap 60 SC060XAK6) connected in series to a 0.2µm bacterial filter from Pall (Mini kleenpak™ 20, réf. KM5EKVP2S).

Conclusion: the combination of the use of 1% TnBP-1% Triton X-45 followed by 1 extraction by soybean oil allows obtaining over 350 ml of clear recovered plasma that can be filtered by gravity through a charcoal filter and a bacterial filter to eliminate the SD agents oil, residues, blood cell debris, bacteria and parasites.

### Example 12:

### Recovered plasma treated with 1% TnBP-1% Triton X-45 (reference 93419, Fluka, Sigma Aldrich) and subjected to 1 extraction by soybean oil (ref. 19-42500, Penta International Corporation)

2 units of recovered plasma were prepared from whole blood, collected into CPD anticoagulant/preservative (ratio: 14ml/100 ml of blood). Blood was centrifuged at 3600 g for 12 min within 4hrs of collection.Plasma was not leucoreduced prior to processing. Plasma was frozen in a dry ice ethanol bath and stored in a freezer at-30°C for a maximum of 3 months.

The 2 plasma donations were thawed in a temperature-controlled water-bath at 35°C for 10 min and then pooled into the first SD treatment bag. The volume of the plasma pool was 400ml.

8 ml of a 50%/50% mixture of TnBP (Prolabo, VWR, Fontenay-sous-bois, France) and Triton X-45 (reference 93419, Fluka, Sigma Aldrich) was carefully added to the 400 ml of plasma to reach final concentrations of 1% TnBP and 1% Triton X-45.

The plasma-TnBP-Triton X-45 mixture was shaken vigorously for 5 min to ensure good mixing of the various components. The processing bag was then incubated in a shaker-incubator at 31°C and then treated for 30 minutes under constant gentle stirring (150 rpm). The plasma-TnBP-Triton X-45 mixture was then transferred into the second SD treatment bag and incubated in the same conditions for 1h30min.

The mixture was then transferred to the oil extraction bag. 40 ml of soybean oil (ref. 19-42500, Penta International Corporation, USA) was added to the plasma-TnBP-Triton X-45 mixture. The plasma-TnBP-Triton X-45/ 10 vol% oil suspension was shaken vigorously for at least 1 min and then put onto the shaker-incubator to ensure gentle stirring for at least 15 min. The bag was then suspended to ensure 45 minutes decantation between the oil phase (upper layer) and the plasma phase (lower layer).

A clear plasma phase was recovered. Over 370ml of plasma passed through an infusion filter and then could be filtered by gravity (without pressure applied by a pump) through a AKS6 Pall charcoal filter (SUPRAcap 60 SC060XAK6) connected in series to a 0.2µm bacterial filter from Pall (Mini kleenpak™ 20, réf. KM5EKVP2S).

Conclusion: the combination of the use of 1% TnBP-1% Triton X-45 followed by 1 extraction by soybean oil allows obtaining over 350 ml of a clear plasma that can be filtered by gravity through a charcoal filter and a bacterial filter to eliminate the SD agents oil residues, blood cell debris, bacteria and parasites.

### Example 13:

### Apheresis plasma treated with 1% TnBP-1% Triton X-45 (X45, Triton® X-45, Sigma) and not subjected to soybean oil extraction

2 plasma units were collected by apheresis procedures following manufacturers' instructions and anticoagulated with ACDA (ratio: 8ml/100 ml of blood). Plasma was not leucoreduced prior to processing. Plasma was frozen in a dry ice ethanol bath and stored in a freezer at -30°C for a maximum of 3 months.

The 2 plasma donations were thawed in a temperature-controlled water-bath at 35°C for 10 min and then pooled into the first SD treatment bag. The volume of the plasma pool was 400ml.

8 ml of a 50%/50% mixture of TnBP (Prolabo, VWR, Fontenay-sous-bois, France) and Triton X-45 (X45, Triton® X-45, Sigma) was carefully added to the 400 ml of plasma to reach final concentrations of 1% TnBP and 1% Triton X-45.

The plasma-TnBP-Triton X-45 mixture was shaken vigorously for 5 min to ensure good mixing of the various components. The processing bag was then incubated in a shaker-incubator at 31°C and then treated for 30 minutes under constant gentle stirring (150 rpm). The plasma-TnBP-Triton X-45 mixture was then transferred into the second SD treatment bag and incubated in the same conditions for 1h30min.

A very turbid plasma phase was recovered that could not be filtered through a AKS6 Pall charcoal filter (SUPRAcap 60 SC050XAK6).

Conclusion: the extraction with soybean oil after 1% TnBP-1% Triton X-45 is needed to clarify the plasma and make the charcoal filtration possible.

Table 1 hereafter shows a comparison of the quality of plasma treated as described in Example 1 (labelled "Final") compared the respective starting plasma (labelled "Start") for five batches. Results show that the process using 1% TnBP-1% Triton X-45 followed by 1 soybean oil extraction and by the filtration process on a AKS6 charcoal filter and a bacterial filter does not alter the protein quality of plasma and does not lead to a modification of the ion balance. The TnBP and the Triton X-45 contents are reduced to undetectable levels in the final plasma following 1 oil extraction and charcoal filtration.

**Table 1**

| **Example** | **PH** | **osmolality mosm** | **Ca mg/dl** | **Mg mg/dl** | **Cu mg/dl** | **Fe mg/dl** | **FII %** | **FV %** | **FVIII %** | **FIX %** | **FX %** | **FXI %** | **PC %** | **PS %** | **PT sec** | **aPTT sec** | **TnBP ppm** | **Triton X-45, ppm** |
|---|---|---|---|---|---|---|---|---|---|---|---|---|---|---|---|---|---|---|
| | | | | | | | | | | | | | | | | | | |
| 1 Start | 7,547 | 331 | 2,5 | 1,6 | 49,7 | 40 | 131,9 | 104,7 | 55,5 | 118,1 | 139,7 | 84,9 | 98,4 | 22,6 | 13.2 | 30.5 | NA | NA |
| 1 Final | 7,25 | 322 | 2,2 | 1,6 | 16,7 | 30 | 134,4 | 102,8 | 48,5 | 103,3 | 134 | 61,3 | 122 | 45,9 | 14 | 34.7 | <10* | < 10* |
| 2 Start | 7,49 | 317 | 3,3 | 1,5 | 11 | 31 | 129,4 | 110,8 | 60,5 | 110,8 | 134 | 79,2 | 107,8 | 20,1 | 13.5 | 32.2 | NA | NA |
| 2 Final | 7,52 | 315 | 2,5 | 1,5 | 32 | 47 | 139,6 | 112,9 | 66,8 | 103,3 | 132,6 | 73,2 | 113,9 | 60,7 | 12.5 | 31.3 | <10* | < 10* |
| 3 Start | 7,65 | 351 | 2,7 | 1,6 | 7,6 | 59 | 134,4 | 157,4 | 70,6 | 119 | 144,3 | 81 | 90,9 | 36,1 | 14.2 | 35.3 | NA | NA |
| 3 Final | 7,5 | 307 | 2,5 | 1,5 | 22,4 | 45 | 122,5 | 121,8 | 66,1 | 102,5 | 122,1 | 69,2 | 118,5 | 44.8 | 13.4 | 33.8 | <10* | < 10* |
| 4 Starts | 7,59 | 320 | 2,8 | 1,6 | 39 | 59 | 122,5 | 124,1 | 83,8 | 119 | 111,6 | 78,3 | 79,6 | 34 | 14.8 | 28.4 | NA | NA |
| 4 Final | 7,259 | 324 | 6,8 | 5,1 | 5,7 | 188 | 116 | 104,7 | 75,6 | 97,2 | 101,4 | 71,5 | 84 | 72,7 | 16.5 | 38.3 | <10* | < 10* |
| 5 Start | 7,462 | 307 | 2,9 | 1,3 | 37 | 57 | 114 | 95,6 | 67,5 | 99,4 | 107,4 | 67,7 | 53,3 | 34,4 | 14.8 | 32.6 | NA | NA |
| 5 Final | 7,4 | 318 | 3,3 | 1,6 | 15,2 | 31 | 124,7 | 124,7 | 85,8 | 115,3 | 109,5 | 82 | 72 | 46,7 | 15.8 | 30.3 | <10* | < 10* |
| | | | | | | | | | | | | | | | | | | |
| **Mean Start +/- St Dev** | **7.548 ± 0.03** | **325.2 ± 7.5** | **2.84 ± 0.13** | **1.52 ± 0.06** | **28.86 ± 8.3** | **49.2 ± 5.8** | **126.4 ± 3.7** | **118.5 +/- 10.8** | **67.6 ± 4.8** | **113.3 ± 3.8** | **127.4 ± 7.5** | **78.2 ± 2.9** | **86 ± 9.4** | **29.4 ± 3.3** | **14.1 ± 0.6** | **31.8 ± 2.3** | | |
| **Mean Final +/- St Dev** | **7.385 ± 0.06** | **317.2 ± 3.0** | **3.46 ± 0.85** | **1.59 ± 0.71** | **18.4 ± 4.3** | **68.2 ± 30.2** | **127.4 ± 4.2** | **113.4 +/- 4.4** | **68.6 ± 6.2** | **104.3 ± 3.0** | **119.9 ± 6.4** | **71.4 ± 3.3** | **102.1 ± 10.1** | **54.2 ± 5.5** | **14.4 ± 1.5** | **33.6 ± 2.8** | | |
| P value | P ≤ 0.05 | NS | NS | NS | NS | NS | NS | NS | NS | NS | NS | NS | NS | P ≤ 0.01 | NS | NS | | |

| | | | | | | | | | | | | | | | | | | |
|---|---|---|---|---|---|---|---|---|---|---|---|---|---|---|---|---|---|---|
| St Dev: Standard deviation; NS: non significant difference; NA: not applicable,* after charcoal filtration | | | | | | | | | | | | | | | | | | |

### Example 14:

### Minipool of cryoprecipitate from apheresis plasma treated with 1% TnBP-1% Triton X-45 (X45, Triton® X-45, Sigma) and subjected to 1 extraction by soybean oil (reference S7381, Sigma)

Plasma was collected by apheresis procedures using Baxter (Baxter Healthcare Corp, Deerfield, USA) autopheresis C Blood Cell Separator following manufacturers' instructions. Plasma was anticoagulated with ACDA solution (ratio: 8ml/100 ml of blood).

Cryoprecipitate was prepared by thawing frozen plasma at 4°C overnight until the material becomes slushy. The plasma bags were then centrifuged (model KR4i, Jouan, St Herblain, France) for 30 min at 3850 x g and -4°C. Upon completion of the centrifugation, the bags were put upside down to expel essentially all the cryo-poor plasma to obtain a "dry" cryoprecipitate.

The cryoprecipitate collection bags were then immediately frozen at -20°C or below, for a maximum of 2 months.

On the day of viral inactivation treatment, cryoprecipitate donations were thawed in their collection bags in a temperature-controlled water-bath at 35 +/- 1.5 °C for 10 min.

8 ml of sterile glucose/saline solution was aseptically added to each unit and each bag was mixed manually to fully resuspend the cryoprecipitate. Cryoprecipitate solutions (about 10 ml/bag) were pooled into groups of 40 donations into the first SD treatment bag. The volume of the cryoprecipitate pool was 400ml.

8 ml of a 50%/50% mixture of TnBP (Prolabo, VWR, Fontenay-sous-bois, France) and Triton X-45 (reference 93419, Fluka, Sigma Aldrich) was carefully added to the 400 ml of cryoprecipitate to reach final concentrations of 1% TnBP and 1% Triton X-45.

The cryoprecipitate-TnBP-Triton X-45 mixture was shaken vigorously for 5 min to ensure good mixing of the various components. The processing bag was then incubated in a shaker-incubator at 31°C and then treated for 30 minutes under constant gentle stirring (150 rpm). The cryoprecipitate-TnBP-Triton X-45 mixture was then transferred into the second SD treatment bag and incubated in the same conditions for 1h30min.

The mixture was then transferred to the oil extraction bag. 40 ml of soybean oil (reference S7381, Sigma) was added to the cryoprecipitate-TnBP-Triton X-45 mixture. The cryoprecipitate-TnBP-Triton X-45/ 10 vol% oil suspension was shaken vigorously for at least 1 min and then put onto the shaker-incubator to ensure gentle stirring for at least 15 min. The bag was then suspended to ensure 45 minutes decantation between the oil phase (upper layer) and the cryoprecipitate phase (lower layer).

A clear cryoprecipitate phase was recovered. Over 370ml of cryoprecipitate passed through an infusion filter and then could be filtered by gravity (without pressure applied by a pump) through a Cuno BioCap 30 BC0030 A R32SP filter and then to a 0.2µm bacterial filter from Pall (Mini kleenpak™ 20, réf. KM5EKVP2S).

Conclusion: the combination of the use of 1% TnBP-1% Triton X-45 followed by 1 extraction by soybean oil allows to obtain over 350 ml of a clear cryoprecipitate produced from apheresis plasma that can be filtered by gravity through a charcoal filter and a bacterial filter to eliminate the SD agents oil residues, blood cell debris, bacteria and parasites.

### Example 15:

### Minipool of cryoprecipitate from recovered plasma treated with 1% TnBP-1% Triton X-45 (X45, Triton® X-45, Sigma) and subjected to 1 extraction by soybean oil (ref. 19-42500, Penta International Corporation)

Recovered plasma was obtained from whole blood collected into CPD anticoagulant/preservative (ratio: 14ml/100 ml of blood). Blood was centrifuged at 3600 g for 12 min within 4hrs of collection. Plasma was not leucoreduced prior to processing. Plasma was frozen in a dry ice ethanol bath and stored in a freezer at -30°C for a maximum of 3 months.

Cryoprecipitate was prepared by thawing frozen plasma at 4°C overnight until the material becomes slushy. The plasma bags were then centrifuged (model KR4i, Jouan, St Herblain, France) for 30 min at 3850 x g and -4°C. Upon completion of the centrifugation, the bags were put upside down to expel essentially all the cryo-poor plasma to obtain a "dry" cryoprecipitate.

The cryoprecipitate collection bags were then immediately frozen at -20°C or below, for a maximum of 2 months.

On the day of viral inactivation treatment, cryoprecipitate donations were thawed in their collection bags in a temperature-controlled water-bath at 35 +/- 1.5 °C for 10 min.

8 ml of sterile glucose/saline solution was aseptically added to each unit and each bag was mixed manually to fully resuspend the cryoprecipitate. Cryoprecipitate solutions (about 10 ml/bag) were pooled into groups of 40 donations into the first SD treatment bag. The volume of the cryoprecipitate pool was 400ml.

8 ml of a 50%/50% mixture of TnBP (Prolabo, VWR, Fontenay-sous-bois, France) and Triton X-45 (ref. 19-42500, Penta International Corporation) was carefully added to the 400 ml of cryoprecipitate to reach final concentrations of 1% TnBP and 1% Triton X-45.

The cryoprecipitate-TnBP-Triton X-45 mixture was shaken vigorously for 5 min to ensure good mixing of the various components. The processing bag was then incubated in a shaker-incubator at 31°C and then treated for 30 minutes under constant gentle stirring (150 rpm). The cryoprecipitate-TnBP-Triton X-45 mixture was then transferred into the second SD treatment bag and incubated in the same conditions for 1h30min.

The mixture was then transferred to the oil extraction bag. 40 ml of soybean oil (reference S7381, Sigma) was added to the cryoprecipitate-TnBP-Triton X-45 mixture. The cryoprecipitate-TnBP-Triton X-45/ 10 vol% oil suspension was shaken vigorously for at least 1 min and then put onto the shaker-incubator to ensure gentle stirring for at least 15 min. The bag was then suspended to ensure 45 minutes decantation between the oil phase (upper layer) and the cryoprecipitate phase (lower layer).

A clear cryoprecipitate phase was recovered. Over 370ml of cryoprecipitate passed through an infusion filter and then could be filtered by gravity (without pressure applied by a pump) through a Cuno BioCap 30 BC0030 A R32SP filter and then to a 0.2µm bacterial filter from Pall (Mini kleenpak™ 20, réf. KM5EKVP2S).

Conclusion: the combination of the use of 1% TnBP-1% Triton X-45 followed by 1 extraction by soybean oil allows to obtain over 350 ml of a clear cryoprecipitate produced from recovered plasma that can be filtered by gravity through a charcoal filter and a bacterial filter to eliminate the SD agents oil residues, blood cell debris, bacteria and parasites.

### Example 16:

### Minipool of cryoprecipitate from recovered plasma treated with 1% TnBP-1% Triton X-45 (X45, Triton® X-45, Sigma) and subjected to 1, 2, or 3 extraction by castor oil ((ref. 83912, Fluka)

Recovered plasma was obtained from whole blood collected into CPD anticoagulant/preservative (ratio: 14ml/100 ml of blood). Blood was centrifuged at 3600 g for 12 min within 4hrs of collection. Plasma was not leucoreduced prior to processing. Plasma was frozen in a dry ice ethanol bath and stored in a freezer at -30°C for a maximum of 3 months.

Cryoprecipitate was prepared by thawing frozen plasma at 4°C overnight until the material becomes slushy. The plasma bags were then centrifuged (model KR4i, Jouan, St Herblain, France) for 30 min at 3850 x g and -4°C. Upon completion of the centrifugation, the bags were put upside down to expel essentially all the cryo-poor plasma to obtain a "dry" cryoprecipitate.

The cryoprecipitate collection bags were then immediately frozen at -20°C or below, for a maximum of 2 months.

On the day of viral inactivation treatment, cryoprecipitate donations were thawed in their collection bags in a temperature-controlled water-bath at 35 +/- 1.5 °C for 10 min.

8 ml of sterile glucose/saline solution was aseptically added to each unit and each bag was mixed manually to fully resuspend the cryoprecipitate. Cryoprecipitate solutions (about 10 ml/bag) were pooled into groups of 40 donations into the first SD treatment bag. The volume of the cryoprecipitate pool was 400ml.

8 ml of a 50%/50% mixture of TnBP (Prolabo, VWR, Fontenay-sous-bois, France) and Triton X-45 (ref. 19-42500, Penta International Corporation) was carefully added to the 400 ml of cryoprecipitate to reach final concentrations of 1% TnBP and 1% Triton X-45.

The cryoprecipitate-TnBP-Triton X-45 mixture was shaken vigorously for 5 min to ensure good mixing of the various components. The processing bag was then incubated in a shaker-incubator at 31°C and then treated for 30 minutes under constant gentle stirring (150 rpm). The cryoprecipitate-TnBP-Triton X-45 mixture was then transferred into the second SD treatment bag and incubated in the same conditions for 1h30min.

The mixture was then transferred to the oil extraction bag. 40 ml of soybean oil (reference S7381, Sigma) was added to the cryoprecipitate-TnBP-Triton X-45 mixture. The cryoprecipitate-TnBP-Triton X-45/ 10 vol% oil suspension was shaken vigorously for at least 1 min and then put onto the shaker-incubator to ensure gentle stirring for at least 15 min. The bag was then suspended to ensure 45 minutes decantation between the oil phase (upper layer) and the cryoprecipitate phase (lower layer).

The cryoprecipitate phase recovered was turbid making filtration through a charcoal filter (Cuno BioCap 30 BC0030 A R32SP filter) impossible.

The oil extraction procedure was repeated twice. In each case the cryoprecipitate was turbid making the above-mentioned filtration step impossible.

Conclusion: the combination of the use of 1% TnBP-1% Triton X-45 followed by 1 extraction by castor oil leads to turbid cryoprecipitate produced from recovered plasma that cannot be filtered by gravity through a charcoal filter - bacterial filter sequence.

### Example 17:

### Minipool of cryoprecipitate from apheresis plasma treated with 2% TnBP and subjected to 1, 2, or 3 extractions by soybean oil (reference S7381, Sigma)

40 plasma units were collected by apheresis procedures following manufacturers' instructions and anticoagulated with ACDA (ratio: 8ml/100 ml of blood). Plasma was not leucoreduced prior to processing. Plasma was frozen in a dry ice ethanol bath and stored in a freezer at -30°C for a maximum of 3 months.

Cryoprecipitate was prepared by thawing the 40 frozen plasma units at 4°C overnight until the material becomes slushy. The plasma bags were then centrifuged (model KR4i, Jouan, St Herblain, France) for 30 min at 3850 x g and -4°C. Upon completion of the centrifugation, the bags were put upside down to expel essentially all the cryo-poor plasma to obtain a "dry" cryoprecipitate.

The cryoprecipitate collection bags were then immediately frozen at -20°C or below, for a maximum of 2 months.

On the day of viral inactivation treatment, cryoprecipitate donations were thawed in their collection bags in a temperature-controlled water-bath at 35 +/- 1.5 °C for 10 min.

8 ml of sterile glucose/saline solution was aseptically added to each unit and each bag was mixed manually to fully resuspend the cryoprecipitate. Cryoprecipitate solutions (about 10 ml/bag) were pooled into groups of 40 donations into the first SD treatment bag. The volume of the cryoprecipitate pool was 400ml.

8 ml of TnBP (Prolabo, VWR, Fontenay-sous-bois, France) was carefully added to the 400 ml of cryoprecipitate to reach final concentrations of 2% TnBP.

The cryoprecipitate-TnBP mixture was shaken vigorously for 5 min to ensure good mixing of the various components. The processing bag was then incubated in a shaker-incubator at 37°C and then treated for 30 minutes under constant gentle stirring (150 rpm). The cryoprecipitate-TnBP mixture was then transferred into the second SD treatment bag and incubated in the same conditions for 1h30min.

The mixture was then transferred to the oil extraction bag. 40 ml of soybean oil (reference S7381, Sigma) was added to the cryoprecipitate-TnBP mixture. The cryoprecipitate-TnBP/ 10 vol% oil suspension was shaken vigorously for at least 1 min and then put onto the shaker-incubator to ensure gentle stirring for at least 15 min. The bag was then suspended to ensure 45 minutes decantation between the oil phase (upper layer) and the cryoprecipitate phase (lower layer).

The cryoprecipitate phase recovered was very turbid making filtration through a charcoal filter (Cuno BioCap 30 BC0030 A R32SP filter) impossible.

The oil extraction procedure was repeated twice. In each case the cryoprecipitate was very turbid making the above-mentioned filtration step impossible.

Conclusion: the combination of the use of 2% TnBP followed by 1, 2, or 3 extractions by soybean oil yields in each case in very turbid cryoprecipitate that cannot be filtered by gravity through a Cuno BioCap 30 BC0030 A R32SP filter and even less through a bacterial filter.

### Example 18:

### Minipool of cryoprecipitate from apheresis plasma treated 1% TnBP-1% Triton X-100 and subjected to 1, 2, or 3 extractions by soybean oil (reference S7381, Sigma)

40 plasma units were collected by apheresis procedures following manufacturers' instructions and anticoagulated with ACDA (ratio: 8ml/100 ml of blood). Plasma was not leucoreduced prior to processing. Plasma was frozen in a dry ice ethanol bath and stored in a freezer at -30°C for a maximum of 3 months.

Cryoprecipitate was prepared by thawing the 40 frozen plasma units at 4°C overnight until the material becomes slushy. The plasma bags were then centrifuged (model KR4i, Jouan, St Herblain, France) for 30 min at 3850 x g and -4°C. Upon completion of the centrifugation, the bags were put upside down to expel essentially all the cryo-poor plasma to obtain a "dry" cryoprecipitate.

The cryoprecipitate collection bags were then immediately frozen at -20°C or below, for a maximum of 2 months.

On the day of viral inactivation treatment, cryoprecipitate donations were thawed in their collection bags in a temperature-controlled water-bath at 35 +/- 1.5 °C for 10 min.

8 ml of sterile glucose/saline solution was aseptically added to each unit and each bag was mixed manually to fully resuspend the cryoprecipitate. Cryoprecipitate solutions (about 10 ml/bag) were pooled into groups of 40 donations into the first SD treatment bag. The volume of the cryoprecipitate pool was 400ml.

8 ml of 50%/50% mixture of TnBP (Prolabo, VWR, Fontenay-sous-bois, France) and Triton X-100 (Merck, Darmstadt, Germany) was carefully added to the 400 ml of cryoprecipitate to reach final concentrations of 1% TnBP-1% Triton X-100.

The cryoprecipitate-TnBP-Triton X-100 mixture was shaken vigorously for 5 min to ensure good mixing of the various components. The processing bag was then incubated in a shaker-incubator at 37°C and then treated for 30 minutes under constant gentle stirring (150 rpm). The cryoprecipitate- TnBP-Triton X-100 mixture was then transferred into the second SD treatment bag and incubated in the same conditions for 1h30min.

The mixture was then transferred to the oil extraction bag. 40 ml of soybean oil (reference S7381, Sigma) was added to the cryoprecipitate- TnBP-Triton X-100 mixture. The cryoprecipitate- TnBP-Triton X-100/ 10 vol% oil suspension was shaken vigorously for at least 1 min and then put onto the shaker-incubator to ensure gentle stirring for at least 15 min. The bag was then suspended to ensure 45 minutes decantation between the oil phase (upper layer) and the cryoprecipitate phase (lower layer).

The cryoprecipitate phase recovered was turbid and could not be filtered through the AKS6 Pall charcoal filter (SUPRAcap 60 SC060XAK6)

The oil extraction procedure was repeated twice. In each case the cryoprecipitate was very turbid making the above-mentioned filtration step impossible.

Conclusion: the combination of the use of 1% TnBP-1% Triton X-100 followed by 1, 2, or 3 extractions by soybean oil yields in each case a turbid cryoprecipitate that cannot be filtered by gravity through a Cuno BioCap 30 BC0030 A R32SP filter and even more so through a bacterial filter.

### Example 19:

### Minipool of cryoprecipitate from apheresis plasma treated with 1% TnBP-1% Triton X-45 (X45, Triton® X-45, Sigma) and subjected to 1 extraction by soybean oil (reference S7381, Sigma) and to filtration on Cuno BioCap 25 R32SP

Cryoprecipitate was processed as described in Example 14, but the activated charcoal filter used was a Cuno BioCap 25 BC0025 L R32SP

Over 380ml of cryoprecipitate passed through an infusion filter and then could be filtered by gravity (without pressure applied by a pump) through this Cuno BioCap 25 BC0025 L R32SP filter and then to a 0.2µm bacterial filter from Pall (Mini kleenpak™ 20, réf. KM5EKVP2S).

Conclusion: the combination of the use of 1% TnBP-1% Triton X-45 followed by 1 extraction by soybean oil allows to obtain over 350 ml of a clear cryoprecipitate produced from apheresis plasma that can be filtered by gravity through a BioCap 25 BC0025 L R32SP charcoal filter and a bacterial filter to eliminate the SD agents oil residues, blood cell debris, bacteria and parasites.

Table 2 hereafter shows a comparison of the quality of cryoprecipitate treated as described in Example 14 (labelled "Final") compared the respective starting cryoprecipitate (labelled "Start") for two batches. Results show that the process using 1% TnBP-1% Triton X-45 followed by 1 soybean oil extraction and by the filtration process on the Cuno BioCap 30 BC0030 A R32SP and the bacterial filter do not alter the protein quality of cryoprecipitate. The TnBP and the Triton X-45 are reduced to undetectable levels in the final cryoprecipitate following 1 oil extraction and charcoal filtration.

**Table 2**

| | Factor VIII | Clottable Fibrinogen, g/l | TnBP | Triton X-45 |
|---|---|---|---|---|
| | IU | | Ppm | Ppm |
| 1 Start | 8.5 | 16.9 | NA | NA |
| 1 Final | 8.0 | 15.7 | < 10ppm | < 15ppm |
| 2 Start | 7.5 | 16.2 | NA | NA |
| 2 Final | 7.3 | 16.8 | < 10ppm | < 15ppm |

| | | | | |
|---|---|---|---|---|
| NA: not applicable, * after charcoal filtration | | | | |

Table 3 hereafter shows a comparison of the quality of cryoprecipitate treated as described in Example 19 (labelled "Final") compared the respective starting cryoprecipitate (labelled "Start") for one batch. Results show that the process using 1% TnBP-1% Triton X-45 followed by 1 soybean oil extraction and by the filtration process on the Cuno BioCap 25 BC0025 L R32SP and the bacterial filter do not alter the protein quality of cryoprecipitate.

**Table 3**

| Factor | Factor VIII | Clottable Fibrinogen, g/l |
|---|---|---|
| | IU | |
| 1 Start | 7.3 | 13.6 |
| 1 Final | 7.0 | 13.5 |

### Example 20

### Plasma treated with 1 % TnBP-1 % Triton X-45 and subjected to 1 extraction by soybean oil

Plasma was collected at the Shabrawishi hospital blood bank (Giza, Cairo, Egypt) from regular volunteer plasma donors who provided informed consent. Plasma was obtained by apheresis procedures or recovered from whole blood, and frozen at -20°C or below, as described in Burnouf et al., TRANSFUSION, Volume 46, December 2006, 2100-8.

2 plasma donations were thawed in a temperature-controlled water-bath at 35°C for 10 min and then pooled into the first SD treatment bag. The volume of the plasma pool was 400ml.

The plasma pool was treated in a bag system according to figure 1 (without transfusion filter, but comprising an additional pooling bag between the bacterial filter and the collection bags) with a 50%/50% mixture of TnBP (Prolabo, VWR, Fontenay-sous-bois, France) and Triton X-45 (reference 93419, Fluka, Sigma Aldrich) followed by extraction of the process chemicals with soybean oil as described in Example 1.

A clear plasma phase was recovered. Over 370ml was filtered by gravity (without pressure applied by a pump) through a AKS6 Pall charcoal filter (SUPRAcap 60 SC060XAK6) connected in series to a 0.2µm bacterial filter from Pall (Mini kleenpak™ 20, réf. KM5EKVP2S).

The filtered plasma was mixed in a pooling bag and then dispensed into two individual 200-mL bags. All bags are separated by breakable valves to ensure the segregation between each step. Once used, bags are cut from the system and disposed.

### EXAMPLE 21

### Minipool of cryoprecipitate treated with 1% TnBP-1% Triton X-45 and subjected to 1 extraction by soybean oil

Cryoprecipitate was prepared by thawing frozen plasma at 4°C overnight until the material becomes slushy. After centrifugation (model KR4i, Jouan, St Herblain, France) for 30 min at 3850 x g and -4°C, the CPP was almost completely removed, leaving a low volume cryoprecipitate. The cryoprecipitate collection bags were then immediately frozen at -20°C or below, for a maximum of 2 months.

32-40 low volume-cryoprecipitate units were pooled under laminar flow in a 400-mL bag and subjected to S/D treatment using 1% TnBP (Prolabo, VWR, Fontenay-sous-bois, France) and 1% Triton X-45 (Sigma Chemical, St Louis, MO) at 31°C followed by extraction of the process chemicals with soybean oil as described in Example 15, using a processing system according to figure 2 (without transfusion filter).

The obtained cryoprecipitate is mixed in a pooling bag and then dispensed into several 40-mL bags. All bags are separated by breakable valves to ensure the segregation between each step. Once used, bags are cut from the system and disposed.

### EXAMPLE 23

### Analysis of the plasma and cryoprecipitate obtained in Examples 20 and 21

### 1. Assays

### Blood cell counts and cell marker determinations

Platelets, while blood cells (WBC), and red blood cells counts were determined using a cell counte (Cell Dyn, Abbott). Blood cell antigen markers CD 41 for leucocytes, CD61 for platelets and Glycophorin A for RBCs were determined by flow-cytometry (Becton-Dickinson).

### Protein assays

Multiple 1-ml samples were taken on the starting plasma and cryoprecipitate minipools and on the final pools, frozen at -80°C until protein assessment. Most protein determinations were performed using the procedures described before. Von Willebrand factor ristocetin cofactor (VWF:RCo) and collagen binding (VWF:CB) assays, and VWF multimer electrophoretic separations were done as described before. Factor XIII functional activity was determined by Berichrom FXIII (Siemens),and antithrombin functional activity by Coamatic AT (Chromogenix). All samples were analyzed in homogeneous series comprising the starting and treated plasma materials to limit assay variations.

### Sodium dodecyl sulfate-polyacrylamide gel electrophoresis (SDS-PAGE)

The protein profile of the plasma and cryoprecipitate prior to and after S/D processing was studied by SDS-PAGE under non-reduced and reduced conditions. 0.5µl of unreduced samples were mixed with 2.5µl of NUPAGE LDS 4X (Invitrogen Corporation, Carlsbad, California, USA) sample buffer and 6 µl RIPA, heated for 10 min at 70°C, and separated on 4-12% polyacrylamide gradient gels in a MES (Invitrogen) buffer. Reduced samples were also prepared under the same conditions apart from the fact that 1µl of NuPAGE Reducing Agent (Invitrogen) was added to the mixture. Separation was carried out at constant 200V, initial 150mA/gel, for 35 min. Novex® Sharp Pre-Stained Protein Molecular Weight Standard (Invitrogen) was used for molecular weight assessment: 260, 160, 110, 80, 60, 50, 40, 30, 20, 15, 10, and 3.5KDa. Gels were stained using coomassie blue.

### TnBP and Triton X-45assays

TnBP and Triton X-45 were extracted from plasma and cryoprecipitate samples and analyzed by gas chromatography (GC) and high-performance-liquid chromatography as described before in Burnouf et al, Transfusion 2006; 46 (12):2100-8).

### Di(2-ethylhexyl) phthalate (DEHP) assay

Di(2-ethylhexyl) phthalate (DEHP) was assessed on the starting and final plasma and cryoprecipitate minipools. 1-mL of plasma or cryoprecipitate was mixed with 0.125 ml of 1.5 N perchloric acid (Riedel-de-Haën, Seelze, Germany) in a glass tube and incubated for 15 min at 37 ºC. After the end of the incubation period, the content of the glass tubes was mixed with ice cooled ethanol (Merck, Germany): chloroform (Fisher-Scientific, Illkirch, France) at a rate of 1:1:0.092 ml and incubated for 2 hrs under - 20ºC. The samples were centrifuged at 1000 x g for 30 min and carefully transfer the supernatant to clean glass tube. 4-mL of n-hexane (Merck, Germany) were added to the supernatant and shacked vigorously, then let to settle for 5 min to separate two layers. The upper hexane layer was transferred without disturbing the interface to a clean glass tube. The last step was repeated twice and the hexane portions were collected in the same glass tube. The hexane was evaporated until dryness, then dissolved in 1-mL acetonitrile for HPLC (LabScan, England). DEHP was determined by HPLC (SFD, Schemback GmbH, Germany) equipped with analytical pump (SFD 9414) and UV/VIS detector (S 3210) at a wavelength of 202 nm and Lichrospher 100 RP 18-5µ, (250 mm x 4.6 mm) column (CS-Chromatographie, Germany). A mixture of 85:15 of acetonitrile and methanol (Merk, Germany) was used as mobile phase at 1.5 ml/min using 8 min analysis time.

### Cholesterol content

Cholesterol content was determined using an Enzymatic Colorimetric test, mono reagent with liquid clearing factor (CHOD-PAP), from Human Diagnostic (www.human.de). The assays were read by a Spectrophotometer from Spinreact (Spain) at a wave length 546 and with a sensitivity of 1 mg/dl.

### Toxicity assay

The toxicity of the SD-treated plasma and SD-treated cryoprecipitate (after oil extraction, S/D adsorption filter, and after sterile filtration) was evaluated in Sprague Dawley rats, and compared to untreated plasma and cryoprecipitate and control saline. 36 rats weighing 80 - 100 g were divided into 9 groups of 4 rats, comprising 2 males and 2 females, each. The rats were acclimatized to the experimental environment for 7 days and their weights were recorded. Each rat was injected intravenously with the plasma fractions or saline, at a dose of 6.5 ml/kg body weight. The rats were then observed for 14 days, with daily check for abnormal behavior or clinical signs, body weight, and daily consumption of water and food.

### Statistical analysis

Data for all series of experiments are reported as mean, standard deviation and minimal and maximal values. Statistical comparisons were made with a two-tailed paired Student test. A p value of less than 0.05 was used to assess the significance of the differences in mean PGF concentration between the platelet preparations at different steps of the procedure. Values are presented as non significant (NS; <0.05), < 0.01, or <0.001. Precise p values are indicated when close to 0.05.

### 2. Results

### Plasma

Table 2 hereafter shows the data of the analysis of recovered (A) or apheresis (B) plasma (8 consecutive runs in each group) before and after S/D-F treatment (solvent/detergent treatment followed by soybean oil extraction and filtrations) according to Example 20. Excellent preservation of the characteristics of plasma was observed. There was over 90 to 95% recovery of the functional activity of most coagulation factors and protease inhibitors and anticoagulants, with non-significant differences between the starting plasma and the SD-F plasma. The only significant decrease of activity was observed for FV with 88 and 82% recovery in recovered and apheresis plasma, respectively (p < 0.01), and alpha 2-antiplasmin with 91% recovery in recovered plasma. There was a small, but significant, increase in the PT in both recovered (p <0.01) and apheresis (p <0.05) SD-F plasma and in aPTT (p<0.05) in SD-F apheresis plasma. The content in total protein, albumin, IgG, and IgM was not significantly modified by the treatment. The SD-F plasma met all quality criteria of FFP (fresh frozen plasma).

TnBP and Triton X-45 were less than 10 and 50 ppm in all plasma units indicating the efficiency of the oil extraction process.

### Cryoprecipitate

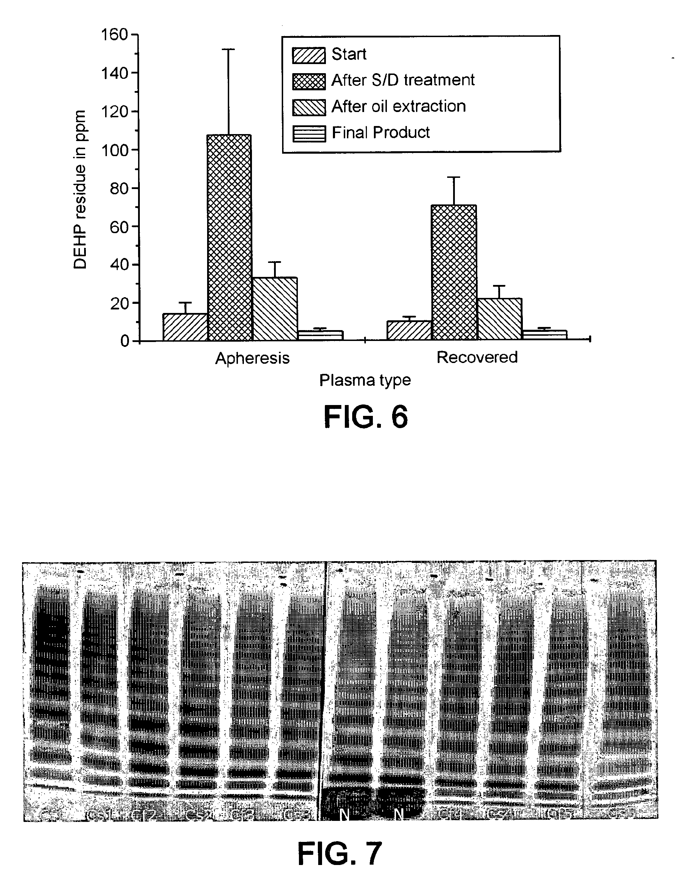

Table 3A shows the data of the analysis of cryoprecipitate minipools before and after the S/D-F treatment (solvent/detergent treatment followed by soybean oil extraction and filtrations) and Fig 7 shows the VWF multimeric profile for the starting cryoprecipitate (lanes Cs1, Cs2, Cs3, Cs4, and Cs5) and the final cryoprecipitate (lanes Cf1, Cf2, Cf3, Cf4, and Cf5) of five different batches as well as for reference plasma (standard plasma from Siemens, lane N). The mean FVIII content in the S/D-F cryoprecipitate minipools was close to 9 IU/mL, and mean fibrinogen close to 15 mg/mL. Mean VWF:Ag, VWF:RCo, and VWF:CB content was 17.5, 14.6, and 13.5 IU/ml, respectively in the S/D-F cryoprecipitate minipools. There was no significant difference with values found in the starting cryoprecipitate minipool. The mean RCo/Ag (0.84) and CBA/Ag (0.87) ratio was not affected by the S/D treatment. The multimeric distribution in the S/D-F cryoprecipitates was not distinguishable from that of the starting cryoprecipitate minipools. The percentage of >15 mers and >10 mers multimers in each individual run, prior to and after S/D-F treatment, and the respective ratios to NP multimers is shown in Table 3. The mean percentage of >15 mers was about 7.8% (corresponding to ratio compared to NP of 0.8) and that of >10 mers multimers between about 23.5 % (respective ratios of 0.92as compared to NP) (not shown). The CB activity and >15 mers content was well correlated. The titer in anti-A and anti-B iso-agglutinins was found to range from 0 - 1, and 0 - 1/8, respectively, in the cryoprecipitate minipools before and after S/D treatments.

### Sodium dodecyl sulfate-polyacrylamide gel electrophoresis (SDS-PAGE)

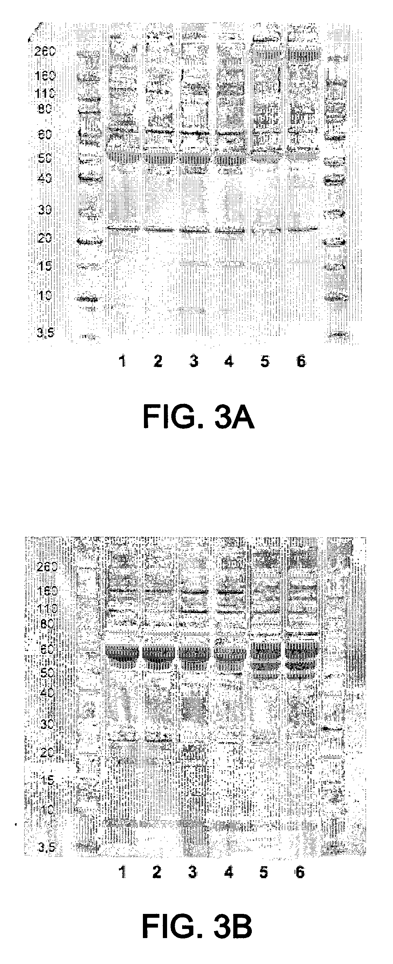

The electrophoretic patterns under unreduced (Fig 3A) and reduced (Fig 3B) conditions did not reveal any detectable differences between the starting plasmas (lanes 1 and 3) and cryoprecipitate minipools (lane 5) and the corresponding S/D-F (solvent/detergent treatment followed by soybean oil extraction and filtrations) treated plasma (lanes 2 and 4) and cryoprecipitate minipool (lane 6).

### TnBP and Triton X-45 content

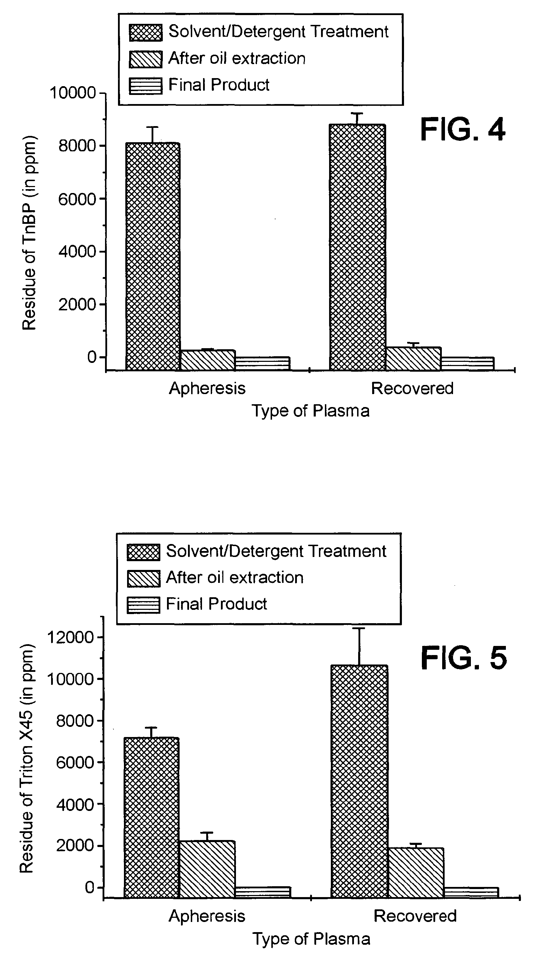

Figures 4 and 5 show the mean content in TnBP and Triton X-45 in the plasma after S/D treatment, after oil extraction and after filtrations. One oil extraction reduced the mean residual TnBP to 261 +/-146 (apheresis plasma) and 393 +/- 495 ppm (recovered plasma), and less than 1 ppm after filtrations (apheresis and recovered plasmas). The respective Triton X-45 residual level was 2248 +/- 1189 and 1906 +/- 660 after the oil extraction and 18 +/- 25 and 12 +/- 14 ppm after filtrations. Similar values were obtained with the cryoprecipitate.

### DEHP

Table 4 shows the results of DEHP determination along the various steps of the processing of apheresis plasma or recovered plasma according to Example 20. The results are also illustrated in Figure 6. The mean content in DEHP, which was 14.3 +/- 16.3 and 9.9 +/-6.6 ppm in starting apheresis and recovered plasma, respectively, increased to 107.7 +/- 140.2 and 70.6 +/-45 after SD treatment. DEHP level in plasma was reduced by both the oil extraction (33.0 +/- 25.5; 21.6 +/-20.1, respectively) and the filtrations (5.1 +/- 3.4; 4.7 +/- 3.9, respectively). Similar values were obtained with the cryoprecipitate.

### Toxicity

There was no significant difference between the mean weight gain in the groups receiving saline (control) and the S/D-F plasma (G8) and cryoprecipitate (G9). Weight gain were higher then that in the groups receiving the untreated plasma and cryoprecipitate. Results are presented in table 5. The indicated variations of body weight are given in grams.

Conclusion: The mean residual level of TnBP and Triton X-45 was less than 10 ppm and less than 50 ppm, respectively, meeting the limits that have been approved for a range of SD-treated plasma derivatives and other biological products. Over 90% and 70?% of the TnBP and Triton X-45 are removed by the oil extraction, and the remaining chemicals were adsorbed on the filter. Such oil extraction / filtration combination has multiple advantages in terms of product quality. First, it reduces DEHP, the plasticizer used in the manufacture of PVC bags, to levels lower than that found in the starting plasma. Second, there is a reduction in the content of cholesterol and triglycerides which clarifies the final products dramatically and may present clinical advantages for the treatment of cholesterolemic patients. The removal of lipids may also have beneficial effects for reducing the risks of TRALI associated to lipids. Third, the pre-clarification of the plasma on the material makes it possible to perform integral online filtration on a 0.2 µm membrane. As a consequence, the end-products are extremely clear and shiny, even if the starting plasma or cryoprecipitate are slightly turbid or opalescent. The 0.2-µm-filtration also ensures a safeguard against potential adventitious bacterial contamination at the time of collection, pooling of the plasma fractions, or during the process. It also removes blood cell debris, as demonstrated here, as well as micro-aggregates or any hypothetical blood-borne intracellular bacteria or parasites that could be transmitted by plasma. To the knowledge of the inventors, this is the first method allowing blood bank plasma components to be successfully subjected to 0.2µm sterile filtration.

**Table 2A : Recovered plasma: biochemical composition before and after S/D treatment, soybean oil extraction and filtrations**

| **Recovered Plasma (N=8)** | | | | |
|---|---|---|---|---|
| | Start | | Final | |
| | Mean | SD* | Mean (% recovery) | SD |
| **Fibrinogen, mg/mL** | **293.4** | 52 | **277.4** (95%) | 54.4 |
| **Factor II, IU/mL** | **0.94** | 0.12 | **0.95** (101%) | 0.14 |
| **Factor V, IU/mL** | **1.27** | 0.17 | **1.12** (88%) | 0.17 |
| **Factor VII,IU/mL** | **1.02** | 0.14 | **0.99** (97%) | 0.13 |
| **Factor VIII, IU/mL** | **1.24** | 0.25 | **1.27** (102%) | 0.26 |
| **Factor IX, IU/mL** | **0.98** | 0.13 | **0.85** (87%) | 0.19 |
| **Factor X, IU/mL** | **0.87** | 0.17 | **0.69** (79%) | 0.20 |
| **Factor XI, IU/mL** | **0.61** | 0.12 | **0.55** (90%) | 0.12 |
| **Factor XIII, IU/mL** | **0.83** | 0.26 | **0.84** (101%) | 0.26 |
| **VWFAg, IU/mL** | **1.12** | 0.21 | **1.11** (99.1%) | 0.21 |
| **VWFRCo, IU/mL** | **1.10** | 0.25 | **1.10** (100%) | 0.31 |
| **Protein S, IU/mL** | **0.86** | 0.23 | **0.96** (112%) | 0.08 |
| **Protein C, IU/mL** | **0.76** | 0.13 | **0.80** (105%) | 0.11 |
| **Antithrombin, IU/mL** | **0.88** | 0.14 | **0.91** (103%) | 0.11 |
| **Alpha 2-antiplasmin, U/mL** | **0.89** | 0.16 | **0.81** (91%) | 0.19 |
| **C3, mg/ml** | **719** | 156 | **686** (95%) | 125 |
| **IgG, mg/ml** | **8.9** | 2.25 | **9.14** (103%) | 1.81 |
| **IgA, mg/ml** | **1 .46** | 0.15 | **1.46** (100%) | 0.20 |
| **IgM, mg/ml** | **1.22** | 0.35 | **1.16** (95%) | 0.28 |
| **Albumin, mg/ml** | **33.2** | 4.8 | **32.3** (97%) | 4.3 |
| **Protein, mg/ml** | **55.4** | 4.8 | **58.7** (106 %) | 8.26 |
| **PT, sec** | **13.8** | 0.5 | **14.3** | 0.7 |
| **aPTT, sec** | **29.6** | 2.5 | **30.9** | 1.7 |
| **Cholesterol, mg/ml** | **84.0** | 13.3 | **n.d.**** | n.d**. |
| **Triglycerides, mg/ml** | **81.8** | 50.1 | **19.9** (24%) | 6.6 |

| | | | | |
|---|---|---|---|---|
| * SD = standard deviation; ** n.d. = not detectable | | | | |

**Table 2B: Apheresis plasma: biochemical composition before and after S/D treatment, soybean oil extraction and filtrations**

| **Apheresis Plasma (N=8)** | | | | |
|---|---|---|---|---|
| | **Start** | | **Final** | |
| | Mean | SD | Mean (% recovery) | SD |
| **Fibrinogen, mg/mL** | **276.5** | 51.1 | **260.2** (94%) | 42.5 |
| **Factor II, IU/mL** | **0.98** | 0.19 | **0.95** (97%) | 0.12 |
| **Factor V, IU/mL** | **1.28** | 0.22 | **1.05** (82%) | 0.25 |
| **Factor VII, IU/mL** | **1.13** | 0.13 | **1.09** (96%) | 0.11 |
| **Factor VIII, IU/mL** | **1.21** | 0.35 | **1.27** (105%) | 0.35 |
| **Factor IX, IU/MI** | **1.09** | 0.20 | **1.04** (95%) | 0.20 |
| **Factor X, IU/mL** | **0.98** | 0.20 | **0.96** (98%) | 0.15 |
| **Factor XI, IU/mL** | **0.80** | 0.18 | **0.77** (96%) | 0.15 |
| **Factor XIII, IU/mL** | **0.77** | 0.22 | **0.75** (97%) | 0.22 |
| **VWFAg, IU/mL** | **1.19** | 0.32 | **1.22** (102%) | 0.33 |
| **VWFRCo, IU/mL** | **1.04** | 0.33 | **1.12** (107%) | 0.33 |
| **Protein S, IU/mL** | **0.75** | 0.20 | **0.94** (125%) | 0.12 |
| **Protein C, IU/mL** | **1.00** | 0.32 | **1.04** (104%) | 0.31 |
| **Antithrombin,IU/mL** | **1.09** | 0.08 | **246** (68%) | 44 |
| **Alpha 2-antiplasmin, U/mL** | **0.98** | 0.15 | **1.03** (94%) | 0.12 |
| **C3, mg/ml** | **779** | 101 | **827** (106%) | 70 |
| **IgG, mg/ml** | **7.23** | 2.09 | **7.07** (98%) | 2.45 |
| **IgA, mg/ml** | **1.05** | 0.32 | **1.02** (97%) | 0.32 |
| **IgM, mg/ml** | **0.97** | 0.26 | **1.05** (108%) | 0.28 |
| **Albumin, mg/ml** | **30.01** | 2.3 | **29.9** (99%) | 2.3 |
| **Protein, mg/ml** | **49.4** | 3.29 | **49.1** (99%) | 3.2 |
| **PT, sec** | **13.31** | 0.93 | **14.21** | 1.6 |
| **aPTT, sec** | **26.4** | 2.1 | **29.4** | 3.9 |
| **Cholesterol, mg/ml** | **105.7** | 7.4 | **3.4** (3%) | 7.95 |
| **Triglycerides, mg/ml** | **116.7** | 47.35 | **23.6** (20%) | 4.9 |

| | | | | |
|---|---|---|---|---|
| SD = standard deviation | | | | |

**Table 3: Cryoprecipitate: biochemical composition before and after S/D treatment, soybean oil extraction and filtrations (Data of 5 consecutive runs)**

| | **Start** | | **Final** | |
|---|---|---|---|---|
| | Mean | SD* | Mean (% recovery) | SD* |
| **Factor VIII, IU/mL** | **8.66** | 0.97 | **9.21 (106%)** | 1.51 |
| **Fibrinogen, mg/mL** | **17.40** | 2.73 | **14.98 (86%)** | 3.66 |
| **Factor XIII, IU/mL** | **2.26** | 0.26 | **2.46 (109%)** | 0.29 |
| **VWF:Ag** | **17.52** | 1.66 | **17.08 (97.5%)** | 1.03 |
| **VWF:RCo** | **13.46** | 2.22 | **14.28 (106%)** | 2.25 |
| **RCo/Ag** | **0.77** | 0.13 | **0.84 (109%)** | 0.14 |
| **VWF/CB** | **14.60** | 3.91 | **14.92 (102%)** | 3.45 |
| **CB/Ag** | **0.83** | 0.16 | **0.87 (105%)** | 0.17 |
| **%> 15 mers** | **7.80** | 1.59 | **7.76 (99.5%)** | 1.34 |
| **> 15 mers / control plasma ratio** | **0.80** | 0.16 | **0.80 (100%)** | 0.14 |
| **%> 10 mers** | **23.82** | 2.02 | **23.54 (99%)** | 1.69 |
| **> 10 mers / control plasma ratio** | **0.93** | 0.08 | **0.92 (99%)** | 0.07 |

| | | | | |
|---|---|---|---|---|
| * SD = standard deviation | | | | |

**Table 4: Determination of DEHP along the various steps of the processing of SD-plasma**

| **Sample type** | **Mean (ppm)** | **± sd** | **No. of Samples** |
|---|---|---|---|
| **Apheresis plasma** | | | |
| Start | 14.34625 | 16.34411 | 8 |
| After SD | 107.753 | 140.2421 | 10 |
| After Oil | 33.0154 | 25.47154 | 10 |
| Final | 5.116 | 3.49165 | 10 |

| **Recovered plasma** | | | |
|---|---|---|---|
| Start | 9.90175 | 6.57638 | 8 |
| After SD | 70.577 | 45.69168 | 10 |
| After Oil | 21.6574 | 20.1105 | 10 |
| Final | 4.707 | 3.9412 | 10 |

| | | | |
|---|---|---|---|
| * SD = standard deviation | | | |

**Table 6: General safety test. Variations in body weight of rats after intravenous infusion of saline (Control); Start plasma (G2); Start cryo (G3); S/D treated-plasma after oil extraction (G4); S/D treated-cryoprecipitate after oil extraction (G5); S/D treated plasma after charcoal filtration (G6); S/D treated cryoprecipitate after charcoal filtration (G7); Final S/D plasma (G8); Final S/D cryoprecipitate (G9)**

| Days Post -treatment | **Control** | **G2** | **G3** | **G4** | **G5** | **G6** | **G7** | **G8** | **G9** |
|---|---|---|---|---|---|---|---|---|---|
| **1** | ± 0,31 | -0,42 ± 3.95 | 2,55 ± 2,76 | 2,22 ± 1,38 | 1,91 ± 0,35 | 2,53 ± 1,10 | 0,20 ± 0,98 | 0,66 ± 2,44 | 2,00 ± 6.77 |
| **2** | 5.15 ± 0,65 | 3,66 ± 3,84 | 5,63 ± 2,39 | 7,00 ± 1,30 | 5,52 ± 0,66 | 4,86 ± 1,91 | 4,54 ± 1,72 | 4,50 ± 3,26 | 7,27 ± 1,19 |
| **3** | 8,56 ± 1,16 | 7,58 ± 4,09 | 8,05 ± 3,21 | 9,62 ± 1,14 | 7,49 ± 2,22 | 6,75 ± 3,86 | 6,62 ± 2,58 | 5,15 ± 2,99 | 11,14 ± 1,86 |
| **4** | 12,77 ± 1,67 | 10,60 ± 3,99 | 12,25 ± 4,11 | 10,93 ± 1,27 | 10,57 ± 2,26 | 10,91 ± 2,85 | 9,31 ± 3,44 | 7,72 ± 3,40 | 14,13 ± 1,53 |
| **5** | 15,75 ± 2,02 | 11,65 ± 3,26 | 15,14 ± 4,36 | 15,19 ± 1,33 | 14,05 ± 2,49 | 15,14 ± 2,62 | 11,40 ± 4,50 | 11,20 ± 4,77 | 16,02 ± 1,95 |
| **6** | 21,25 ± 3,67 | 15,16 ± 3,51 | 16,24 ± 4,41 | 16,16 ± 1,10 | 16,22 ± 2,46 | 15,89 ± 3,11 | 13,69 ± 5,59 | 12,77 ± 5,20 | 19,36 ± 2,34 |
| **7** | 23,36 ± 3,93 | 16,88 ± 4,33 | 19,64 ± 4,50 | 18,73 ± 1,13 | 18,55 ± 3,50 | 20,14 ± 3,43 | 21,31 ± 4,68 | 4,20 ± 3,26 | 21,35 ± 3,44 |
| **8** | 24,75 ± 5,13 | 20,48 ± 4,97 | 22,00 ± 4,17 | 19,29 ± 1,56 | 19,39 ± 4,35 | 23,23 ± 4,16 | 23,14 ± 4,34 | 11,28 ± 5,98 | 23,63 ± 3,56 |
| **9** | 30,42 ± 5,22 | 28,25 ± 4,60 | 24,19 ± 3,67 | 22,64 ± 1,75 | 23.28 ± 4,36 | 26,65 ± 4,09 | 27,17 ± 5,26 | 14.66 ± 5,19 | 26,39 ± 4,34 |
| **10** | 34,75 ± 5,81 | 28,13 ± 4,27 | 27,59 ± 4,61 | 24,31 ± 1,72 | 26,38 ± 5,16 | 28,46 ± 5,44 | 30,34 ± 6,87 | 22,58 ± 2,81 | 32,37 ± 5,92 |
| **11** | 38,00 ± 6,26 | 29,96 ± 5,41 | 30,08 ± 4,55 | 27,17 ± 1,68 | 29,11 ± 6,65 | 32,35 ± 5,10 | 25,20 ± 9,24 | 17,01 ± 6,35 | 32,37 ± 5,92 |
| **12** | 40,58 ± 8,37 | 32,93 ± 6,03 | 31,18 ± 5,41 | 30.10 ± 2,12 | 30,45 ± 7,37 | 36,05 ± 5,65 | 37,07 ± 7,89 | 23,93 ± 4,08 | 34,38 ± 6,65 |
| **13** | 43,28 ± 8,74 | 36,75 ± 6,43 | 34,96 ± 6,00 | 33,54 ± 2.59 | 35,17 ± 7,19 | 40,91 ± 6,30 | 41,36 ± 8,52 | 28,33 ± 3,55 | 40,54 ± 6,88 |
| **14** | 47,65 ± 8,41 | 39,20 ± 7,38 | 35,60 ± 5,64 | 36,34 ± 2,86 | 37,65 ± 7,87 | 49,18 ± 10,77 | 44,68 ± 9,76 | 31,57 ± 3,95 | 43,16 ± 7,00 |

## Claims

1. A non-therapeutic method of virally inactivating a biological fluid selected from the group consisting of mammalian blood, blood plasma, blood serum, plasma fractions, precipitates from blood fractions and supernatants from blood fractionation, platelet poor plasma, platelet-rich plasma, cryo-poor plasma (cryosupernatant), recombinant and transgenic products; the method comprising:
(a) a step of treating the biological fluid with a mixture of tri(n-butyl)phosphate and polyoxyethylene (4-5) p-t-octyl-phenol (t-Oct-C₆H₄-(OCH₂CH₂)ₓOH, x ≈ 5);
(b) an oil extraction step, wherein the tri(n-butyl)phosphate and polyoxyethylene (4-5) p-t-octyl-phenol (t-Oct-C₆H₄-(OCH₂CH₂)ₓOH, x ≈ 5)containing biological fluid is treated with 5 to 15 volume% of soybean oil with respect to the volume of the biological fluid so as to transfer tri(n-butyl)phosphate and polyoxyethylene (4-5) p-t-octyl-phenol (t-Oct-C6H4-(OCH2CH2)xOH, x ≈ 5) from the biological fluid to the soybean oil,
and **characterized in that** it further comprises
(c) passing the biological fluid recovered after step (b) through an activated charcoal filter by gravity; and
(d) passing the biological fluid recovered after step (c) through a bacterial filter by gravity;
provided that the soybean oil extraction step is the sole oil extraction step of the method.

2. The method according to Claim 1, wherein each of the tri(n-butyl)phosphate and the polyoxyethylene (4-5) p-t-octyl-phenol (t-Oct-C₆H₄-(OCH₂CH₂)ₓOH, x ≈ 5) is used in an amount of 1 volume% with respect to the volume of the biological fluid.

3. The method according to Claim 1, wherein the soybean oil is used in an amount of 5 to 15 volume%, preferably of 8 to 12 volume%, and even more preferably about 10 volume% with respect to the volume of the biological fluid.

4. The method according to Claim 1 further comprising, before step (c), a step of passing the biological fluid recovered after step (b) through a transfusion filter.

5. The method according to Claim 4, wherein the filtration through the transfusion filter is carried out by gravity.

6. The method according to Claim 1, wherein the method is carried out using a set of disposable bags (1), comprising a solvent/detergent treatment bag (2) in which step (a) is carried out, and an oil extraction bag (4) in which step (b) is carried out, said second bag having an inner compartment having a funnel-like lower portion that meets an outlet facility of the funnel bag

7. The method according to claim 6, wherein the set of disposable bags further comprises an activated charcoal filter (7) and a bacterial filter (8).

8. Use of the method according to any of Claims 1 to 5 for viral inactivation of a mini-pool of biological fluid.

## Patentansprüche

1. Nicht therapeutisches Verfahren zur viralen Inaktivierung einer biologischen Flüssigkeit, ausgewählt aus der Gruppe bestehend aus Säugetierblut, Blutplasma, Blutserum, Plasmafraktionen, Niederschlägen von Blutfraktionen und Überständen von Blutfraktionierung, plättchenarmem Plasma, plättchenreichem Plasma, cryoarmem Plasma (Cryoniederschlag), rekombinanten und transgenen Produkten; wobei das Verfahren Folgendes umfasst:
(a) einen Schritt des Behandelns der biologischen Flüssigkeit mit einer Mischung aus Tri(n-butyl)phosphat und Polyoxyethylen (4-5) p-t-octyl-phenol (t-Oct-C₆H₄-(OCH₂CH₂)_{X}OH, x ≈ 5);
(b) einen Ölextraktionsschritt, wobei die Tri(n-butyl)phosphat und Polyoxyethylen (4-5) p-t-octyl-phenol (t-Oct-C₆H₄-(OCH₂CH₂)x(OH, x ≈ 5) enthaltende biologische Flüssigkeit mit 5 bis 15 Vol% Soyabohnenöl mit Bezug auf das Volumen der biologischen Flüssigkeit behandelt wird, um Tri(n-butyl)phosphat und Polyoxyethylen (4-5) p-t-octyl-phenol (t-Oct-C₆H₄-(OCH₂CH₂)xOH, x ≈ 5) von der biologischen Flüssigkeit auf das Soyabohnenöl zu übertragen,
und **dadurch gekennzeichnet, dass** es weiter Folgendes umfasst:
(c) Führen der nach Schritt (b) wiedergewonnenen biologischen Flüssigkeit durch einen Aktivkohlefilter durch die Schwerkraft, und
(d) Führen der nach Schritt (c) wiedergewonnenen biologischen Flüssigkeit durch einen bakteriellen Filter durch die Schwerkraft;
vorausgesetzt, das der Schritt der Soyabohnenöl-Extraktion der einzige Schritt der Ölextraktion des Verfahrens ist.

2. Verfahren nach Anspruch 1, wobei jedes von Tri(n-butyl)phosphat und Polyoxyethylen (4-5) p-t-octyl-phenol (t-Oct-C₆H4-(OCH2CH2)xOH, x ≈ 5) .in einer Menge von 1 Vol% mit Bezug auf das Volumen der biologischen Flüssigkeit verwendet wird.

3. Verfahren nach Anspruch 1, wobei das Soyabohnenöl in einer Menge von 5 bis 15 Vol%, vorzugsweise von 8 bis 12 Vol% und noch bevorzugter von ungefähr 10 Vol% mit Bezug auf das Volumen der biologischen Flüssigkeit verwendet wird.

4. Verfahren nach Anspruch 1, weiter umfassend: vor Schritt (c), einen Schritt des Führens der nach Schritt (b) wiedergewonnenen biologischen Flüssigkeit durch einen Transfusionsfilter.

5. Verfahren nach Anspruch 4, wobei die Filtrierung durch den Transfusionsfilter durch die Schwerkraft durchgeführt wird.

6. Verfahren nach Anspruch 1, wobei das Verfahren unter Verwendung eines Satzes von Einwegbeuteln (1) durchgeführt wird, umfassend einen Lösungs-/Reinigungsmittelbehandlungs-Beutel (2), in dem Schritt (a) durchgeführt wird, und einen Ölextraktions-Beutel (4), in dem Schritt (b) durchgeführt wird, wobei der zweite Beutel eine innere Kammer aufweist, die einen trichterförmigen unteren Abschnitt aufweist, der auf eine Auslasseinrichtung des Trichterbeutels trifft.

7. Verfahren nach Anspruch 6, wobei der Satz von Einwegbeuteln weiter einen Aktivkohlefilter (7) und einen bakteriellen Filter (8) umfasst.

8. Verwendung des Verfahrens nach einem der Ansprüche 1 bis 5 zur viralen Inaktivierung eines Minipools von biologischer Flüssigkeit.

## Revendications

1. Procédé non thérapeutique d'inactivation virale d'un liquide biologique choisi dans le groupe constitué de sang de mammifère, plasma sanguin, sérum sanguin, fractions plasmatiques, précipités provenant de fractions sanguines et surnageants provenant du fractionnement sanguin, plasma pauvre en plaquettes, plasma riche en plaquettes, plasma pauvre en cryoprotéines (cryosurnageant), produits recombinants et transgéniques ; le procédé comprenant :
(a) une étape de traitement du liquide biologique avec un mélange de phosphate de tri-n-butyle et de polyoxyéthylène (4-5) p-t-octyl-phénol (t-Oct-C₆H₄-(OCH₂CH₂)ₓOH, x ≈ 5) ;
(b) une étape d'extraction d'huile, dans laquelle le liquide biologique contenant le phosphate de tri-n-butyle et le polyoxyéthylène (4-5) p-t-octyl-phénol (t-Oct-C₆H₄-(OCH₂CH₂)ₓOH, x ≈ 5) est traité avec 5 à 15 % en volume d'huile de soja par rapport au volume du liquide biologique de manière à transférer le phosphate de tri-n-butyle et le polyoxyéthylène (4-5) p-t-octyl-phénol (t-Oct-C₆H4-(OCH2CH2)xOH, x ≈ 5) du liquide biologique à l'huile de soja,
et **caractérisé en ce qu'**il comprend en outre
(c) le passage du liquide biologique récupéré après l'étape (b) à travers un filtre de charbon actif par gravité ; et
(d) le passage du liquide biologique récupéré après l'étape (c) à travers un filtre bactérien par gravité ;
à condition que l'étape d'extraction de l'huile de soja soit la seule étape d'extraction d'huile du procédé.

2. Procédé selon la revendication 1, dans lequel chacun du phosphate de tri-n-butyle et du polyoxyéthylène (4-5) p-t-octyl-phénol (t-Oct-C₆H4-(OCH2CH2)xOH, x ≈ 5) est utilisé dans une quantité de 1 % en volume par rapport au volume du liquide biologique.

3. Procédé selon la revendication 1, dans lequel l'huile de soja est utilisée dans une quantité de 5 à 15 % en volume, de préférence 8 à 12 % en volume, et de manière encore davantage préférée environ 10 % en volume par rapport au volume du liquide biologique.

4. Procédé selon la revendication 1, comprenant en outre, avant l'étape (c), une étape de passage du liquide biologique récupéré après l'étape (b) à travers un filtre de transfusion.

5. Procédé selon la revendication 4, dans lequel la filtration à travers le filtre de transfusion est réalisée par gravité.

6. Procédé selon la revendication 1, dans lequel le procédé est réalisé en utilisant un ensemble de poches jetables (1), comprenant une poche de traitement par solvant/détergent (2) dans laquelle l'étape (a) est réalisée, et une poche d'extraction d'huile (4) dans laquelle l'étape (b) est réalisée, ladite seconde poche comportant un compartiment interne doté d'une partie inférieure de type entonnoir en contact avec un dispositif de sortie de la poche à entonnoir.

7. Procédé selon la revendication 6, dans lequel l'ensemble de poches jetables comprend en outre un filtre de charbon actif (7) et un filtre bactérien (8).

8. Utilisation du procédé selon l'une quelconque des revendications 1 à 5 pour l'inactivation virale d'un groupe de quelques liquides biologiques.
